# FASCICULE DE BREVET EUROPEEN

(11) **EP 3 619 242 B1**
(45) Date de publication et mention de la délivrance du brevet: **30.09.2026**
(21) Numéro de dépôt: 16729957.7
(22) Date de dépôt: 14.04.2016
(51) Int. Cl.: C08B 37/08, C08J 3/075, C12N 5/00, C07K 14/78, C08H 1/00

(54) **PROCÉDÉ DE PRÉPARATION D'HYDROGEL À PARTIR D'ACIDE HYALURONIQUE MODIFIÉ ET DE COLLAGÈNE DE TYPE I**
VERFAHREN ZUR HERSTELLUNG EINES HYDROGELS AUS MODIFIZIERTER HYALURONSÄURE UND TYP-1-KOLLAGEN
METHOD FOR PRODUCING HYDROGEL FROM MODIFIED HYALURONIC ACID AND TYPE 1 COLLAGEN

(30) Priorité: 15.04.2015 FR 1553319
(43) Date de publication de la demande: 11.03.2020
(73) Titulaire: NETRI, 69007 Lyon (FR)
(72) Inventeur: SOUGUIR, Zied, 76100 Rouen (FR); VIDAL, Guillaume, 76000 Rouen (FR); DEMANGE,Elise, 76130 Mont Saint Aignan (FR); LOUIS, Fiona, 35000 Rennes (FR)
(74) Mandataire: IXAS Conseil
(86) Numéro de dépôt international: PCT/FR2016/050863
(87) Numéro de publication internationale: WO 2016/166479

(56) Documents cités:
- WO-A1-2011/161172
- WO-A1-2011/161174
- WO-A1-99/42084
- WO-A2-2009/026158
- WO-A2-2009/116951
- US-A1- 2005 090 008
- CATHERINE A GOUBKO ET AL: "Hydrogel cell patterning incorporating photocaged RGDS peptides", BIOMEDICAL MICRODEVICES, KLUWER ACADEMIC PUBLISHERS, BO, vol. 12, no. 3, 6 March 2010 (2010-03-06), pages 555 - 568, XP019787371, ISSN: 1572-8781
- SHU X Z ET AL: "Attachment and spreading of fibroblasts on an RGD peptide-modified injectable hyaluronan hydrogel", JOURNAL OF BIOMEDICAL MATERIALS RESEARCH, WILEY, NEW YORK, NY, US, vol. 68, no. 2, 1 February 2004 (2004-02-01), pages 365 - 375, XP002526733, ISSN: 0021-9304, DOI: 10.1002/JBM.A.20002
- ELISE DEMANGE ET AL: "Survival of cord blood haematopoietic stem cells in a hyaluronan hydrogel for ex vivo biomimicry", JOURNAL OF TISSUE ENGINEERING AND REGENERATIVE MEDICINE, vol. 7, no. 11, 31 March 2012 (2012-03-31), US, pages 901 - 910, XP055231764, ISSN: 1932-6254, DOI: 10.1002/term.1482

## Description

### Domaine technique de l'invention

L'invention concerne le domaine de la biochimie, et plus particulièrement, le domaine de la biochimie des polysaccharides et des milieux pour culture cellulaire. Plus particulièrement, elle concerne un procédé de préparation d'hydrogel à partir d'acide hyaluronique modifié. Elle concerne également l'utilisation de ces hydrogels comme environnement de cultures cellulaires tridimensionnel.

### Etat de la technique

La majorité des études de culture cellulaire sont effectuées sur des supports bidimensionnels (2D) tels que des plaques de micro-puits et des boîtes de Pétri ; ces dispositifs sont simples et faciles à utiliser, et ils confèrent une viabilité cellulaire élevée aux cellules conservées dans ces supports. Ces systèmes de culture cellulaire 2D ont permis d'améliorer la compréhension de la biologie cellulaire, mais ils ne permettent pas de reproduire les environnements complexes des cultures de cellules *in vivo,* qui sont connus pour moduler la morphologie des cellules, le taux de croissance des cellules, la géométrie de contact, les propriétés de transport des cellules, ainsi que de nombreuses autres fonctions cellulaires.

Les matrices de culture cellulaires tridimensionnelles (3D), appelées par l'homme du métier aussi « scaffolds » (mot anglais signifiant « échafaudages »), ont été mises en place pour surmonter les limites de la culture cellulaire 2D. Ces matrices tridimensionnelles sont des substrats poreux permettant la croissance des cellules, l'organisation, la différenciation cellulaire sur et/ou au sein de leur structure. Il a été démontré que, par rapport aux cultures sur supports bidimensionnels, les supports de culture 3D miment plus ou moins convenablement l'environnement *in vivo* des cellules. Les matrices 3D présentent une diversité architecturale et une diversité de matériels beaucoup plus importantes que celles des substrats 2D. On connaît de nombreux procédés de fabrication de matrices 3D permettant de moduler la porosité, la perméabilité, les caractéristiques mécaniques et la morphologie de surface des matrices, afin de mimer, au mieux, dans un système *in vitro* les divers environnements *in vivo.*

De nombreuses études ont été menées sur l'utilisation de substances naturelles présentes au sein de la matrice extracellulaire (MEC) en tant que support des matrices de culture cellulaires tridimensionnelles.

En effet, la matrice extracellulaire joue un rôle essentiel dans la régulation du comportement des cellules qui entrent en contact avec elle, notamment en modulant le développement cellulaire, la migration, la prolifération, la différenciation, l'adhésion et l'architecture spatiale des cellules et des tissus. La matrice extracellulaire représente l'ensemble des macromolécules extracellulaires des tissus biologiques. Ces macromolécules extracellulaires sont synthétisées et sécrétées par les cellules telles que les ostéoblastes, les chondrocytes ou encore les fibroblastes qui entrent en contact avec la matrice. Ces macromolécules sont ensuite assemblées en une structure tridimensionnelle sous forme d'un maillage 3D dans les espaces extracellulaires de la plupart des tissus. Les macromolécules, présentes dans la matrice extracellulaire, sont principalement des glycoprotéines, des protéines telles que le collagène, et des glycosaminoglycanes (polysaccharides) telles que l'acide hyaluronique.

L'acide hyaluronique est un glycosaminoglycane composé d'un disaccharide de répétition de l'acide glucuronique et de N-acetylglycosamine. L'acide hyaluronique est l'un des polysaccharides biocompatibles et biodégradables les plus répandus dans le corps animal et humain. L'acide hyaluronique, via ses interactions avec les récepteurs membranaires des cellules, participe à un certain nombre d'interactions cellulaires de surface, et contribue ainsi de manière significative à la prolifération, à la migration et à la différenciation cellulaires. De plus, l'acide hyaluronique possède la faculté de former des hydrogels.

Modifier chimiquement l'acide hyaluronique permet de synthétiser des hydrogels possédant des propriétés nouvelles et particulières, comme le soulignent la publication « Chemical modifications of hyaluronic acid for the synthesis of derivatives for a broad range of biomedical applications » par C.E. Schanté et al., parue dans la revue Carbohydrate Polymers, vol. 85, p. 469-489 (2011), la thèse de doctorat de Zied Souguir *(«* Fonctionnalisation de polysaccharides et étude de leurs propriétés 'pH dépendantes' »), Université de Rouen, 2006, et la thèse de doctorat de Kristoffer Bergman « Hyaluronan Derivatives and Injectable Gels for Tissue Engineering », Upsala 2008.

Le collagène peut aussi être employé dans la fabrication de matrices 3D. Le collagène est une protéine fibreuse insoluble présente dans la matrice extracellulaire des organismes. Le collagène contient des acides aminés liés de façon covalente et présente une structure ordonnée en fibrilles qui confère aux tissus une excellente résistance mécanique à la traction. Il existe 28 types de collagènes différents comme le souligne la publication *«* Collagen structure and stability » de M.D Shoulders et R.T. Raines, parue dans l'Annual Review of Biochemistry, vol 78, p929-958 (2009). Le collagène de type I entre dans la constitution de la plupart des tissus conjonctifs tels que le derme, les os, les tendons, les ligaments et la cornée. Les collagènes de type I et VI sont les deux principaux collagènes présents au sein de la matrice extracellulaire adipeuse. Le collagène de type IV se retrouve dans les membranes basales de nombreux tissus, membranes constituées d'une fine couche de matrice extracellulaire séparant les cellules épithéliales des cellules sous'-jacentes.

De nombreux brevets traitent de la réalisation de matériaux à base d'acide hyaluronique et de collagène utilisables comme matrices cellulaires. A titre d'exemple, CN 103 849 594 décrit un procédé de culture *in-vitro* en trois dimensions pour un organe palatin, la matrice employée pouvant être composée d'acide hyaluronique et de collagène. WO 2013/023793 A3 décrit une matrice comprenant un polysaccharide modifié tel que de l'acide hyaluronique et pouvant contenir du collagène. La matrice obtenue peut notamment être utilisée comme « échafaudage » pour la régénération de cellules vivantes. Plusieurs documents, comme par exemple WO 2011/161174 A1, la publication de C.A. Goubko et al. « Hydrogel cell patterning incorporating photocaged RGDS peptides » (Biomedical Microdevices vol. 12 n° 3, p. 555-568 (6 March 2010), ainsi que les demandes de brevet WO 2009/026158 A2 et WO 99/42084, décrivent un hydrogel à base d'acide hyaluronique réticulé pour la culture 3D de cellules. Le document US 2005/090008 décrit l'association entre l'acide hyaluronique et le collagène.

Des matériaux poreux élaborés à partir d'acide hyaluronique et de collagène de type I ont d'ailleurs été développés, comme le présente la publication *«*Studies of novel hyaluronic acid-collagen sponge materials composed of two different species of type I collagen » par Y.K. Lin et D.C Liu, parue dans le journal of biomaterials applications, vol. 21, p265-281 (2007). Cette publication précise d'ailleurs que de nombreuses études ont été réalisées sur ce type de matériaux poreux à base de collagène combiné à de l'acide hyaluronique utilisables comme matériau de reconstruction de la peau ou encore comme matrice cellulaire favorisant la réplication cellulaire pour la reconstruction de tissu.

Certaines macromolécules de la matrice extracellulaire possèdent des sites actifs peptidiques responsables de la fixation des cellules. Le tripeptide RGD (Arg-Gly-Asp) est une séquence d'acides aminés favorisant l'adhésion cellulaire. La séquence tripeptidique RGD est d'ailleurs connue pour ses propriétés d'adhésion cellulaire, notamment de la fibronectine, de la laminine, de l'entactine, du collagène I, du collagène IV et de la ténascine. D'après la fiche technique du tetrapeptide RGDS fournie par la société Cayman Chemical, le motif RGD seul ne permet pas l'adhésion des cellules à la matrice. En revanche, le tétrapeptide RGDS (Arg-Gly-Asp-Ser) permet l'adhésion de plusieurs types cellulaires sur la matrice via notamment les interactions avec les intégrines αVβ1 ou encore αVβ3. D'autres motifs favorisant l'adhésion spécifique de cellules peuvent être employés, tels que le motif galactose favorisant l'adhésion de cellules hépatocytes dans des matrices 3D, comme le précise la publication *«* Galactose-functionnalized PolyHIPE scaffolds for use in routine three dimensional culture of mammalian hepatocytes » de Hayward et al, parue dans la revue Biomacromolecules, vol. 14 p4271-4277 (2013).

WO 2000 04 941 A1 (Pharmacal Biotechnologies) décrit un système de reconstruction osseux, comprenant une matrice pouvant être composée d'hydroxyapatite, une molécule biologiquement active telle qu'une protéine ou un peptide facilitant l'adhésion cellulaire et du collagène.

Bien qu'il existe de nombreuses matrices 3D, les biologistes auront toujours besoin de matrices 3D possédant des propriétés améliorées, notamment d'hydrogels capables de mimer l'environnement biologique de cellules spécifiques et ainsi de présenter une plus grande affinité à leurs regards. Un autre objectif de l'invention est de réaliser par un procédé suffisamment général des matrices 3D adaptables à l'accueil et à la croissance de cellules très différentes.

Un autre objectif de l'invention est de réaliser par un procédé simple et rapide une matrice 3D spécifique qui soit à la fois biocompatible et biodégradable.

Un autre objectif de l'invention est de proposer des matrices 3D spécifiques mimant l'environnement biologique de cellules au regard des principaux constituants (i.e. collagène, acide hyaluronique) de la matrice extracellulaire dont sont issues les cellules d'intérêt.

Un autre objectif de l'invention est de réaliser une matrice 3D spécifique facilement caractérisable.

Un autre objectif de l'invention est de proposer des matrices 3D spécifiques d'une part, de cellules adipocytes et d'autre part de cellules hépatocytes permettant une culture cellulaire 3D satisfaisante.

### Objets de l'invention

La présente invention concerne un procédé de préparation d'un hydrogel à partir d'acide hyaluronique réticulé pouvant être utilisé dans de nombreuses applications impliquant la culture cellulaire 3D. L'hydrogel obtenu peut notamment être employé comme matrice d'une culture cellulaire 3D. En particulier, un procédé selon l'invention permet l'obtention d'un hydrogel d'acide hyaluronique réticulé ayant des propriétés améliorées / optimisées au regard d'un hydrogel d'acide hyaluronique qui n'aurait pas été soumis à un tel traitement. En effet, un hydrogel selon la présente invention est constitué de composants de la matrice extracellulaire, tels que le collagène et l'acide hyaluronique, permettant de mieux imiter l'environnement *in vivo* de certaines cellules. Ainsi, un hydrogel selon l'invention présente une plus grande affinité cellulaire, en particulier pour les cellules hépatocytaires et adipocytaires, de sorte qu'il tend à faciliter l'ingénierie tissulaire.

Un premier objet de l'invention concerne un procédé de fabrication d'un hydrogel lyophilisé comprenant au moins les étapes suivantes, dans lequel :
(a) on fait réagir une fonction amine du groupement peptidique ARG-GLY-ASP-SER (appelé « RGDS ») sur une fonction acide de l'acide hyaluronique, pour obtenir un premier acide hyaluronique au moins partiellement modifié, et
(b) on fait réagir ledit premier acide hyaluronique au moins partiellement modifié avec une solution aqueuse et/ou alcoolique d'un agent réticulant, de préférence de l'acide adipique dihydrazide (ADH), en présence d'au moins un collagène, et de préférence d'au moins un collagène de type I, pour obtenir un hydrogel réticulé,
(c) on lyophilise l'hydrogel réticulé obtenu à l'étape (b); et
(d) on stérilise l'hydrogel lyophilisé obtenu à l'étape (c).

Dans une variante de ce procédé :
(a) on fait réagir une fonction amine du groupement peptidique ARG-GLY-ASP-SER (RGDS) sur une fonction acide de l'acide hyaluronique, pour obtenir un premier acide hyaluronique au moins partiellement modifié, et
(a') on fait réagir une fonction amine du galactosamine (H₂NGal) sur une fonction acide de l'acide hyaluronique, pour obtenir un second acide hyaluronique au moins partiellement modifié,
(b) on fait réagir ledit premier et/ou ledit second acide hyaluronique au moins partiellement modifié avec une solution aqueuse et/ou alcoolique d'un agent réticulant, de préférence de l'acide adipique dihydrazide (ADH) en présence d'au moins un collagène, et de préférence d'au moins un collagène de type I, pour obtenir un hydrogel réticulé,
(c) on lyophilise l'hydrogel réticulé obtenu à l'étape (b); et
(d) on stérilise l'hydrogel lyophilisé obtenu à l'étape (c).

Selon l'invention, à l'étape (a'), le taux de greffage des fonctions acides de l'acide hyaluronique est compris entre 0,5 et 25% molaire, de préférence compris entre 7 et 18%.

Selon l'invention, à l'étape a), le taux de greffage des fonctions acides de l'acide hyaluronique est compris entre 0,5 et 20 % molaire, de préférence 1%.

Selon l'invention, l'étape (a), l'étape (a') et/ou l'étape (b) est (sont) effectuée(s) à un pH compris entre 4,0 et 5,0 , et de préférence à un pH compris entre 4,5 et 4,9 , et encore plus préférentiellement à un pH de 4,75.

Dans un mode de réalisation particulier, l'étape (a), l'étape (a') et/ou l'étape (b) est effectuée en présence d'EDCI et de NHS.

Dans un autre mode de réalisation particulier, le solvant de la solution alcoolique d'un agent réticulant de l'étape (b) est choisi parmi l'éthanol, le propanol, l'isopropanol et le butanol.

Dans un mode de réalisation particulier, le ratio molaire collagène / acide hyaluronique employé à l'étape b) est compris entre 0,1% et 15%.

Dans un mode de réalisation particulier, les collagènes employés à l'étape (b) sont de type I et IV. De préférence, dans ce mode de réalisation particulier, la proportion de collagène de type I est comprise entre 0,1% et 0,4% molaire. De préférence, dans ce mode de réalisation particulier, la proportion de collagène de type IV est comprise entre 0,05% et 0,15% molaire. De préférence, dans ce mode de réalisation particulier, le ratio molaire collagène de type I et IV / acide hyaluronique employé à l'étape b) est compris entre 0,1% et 15%.

Dans un autre mode de réalisation particulier, les collagènes employés à l'étape (b) sont de type I et VI. Avantageusement dans cet autre mode de réalisation particulier, le ratio molaire collagène de type I et VI / acide hyaluronique employé à l'étape b) est compris entre 0,1% et 15%.

Selon l'invention, l'étape (d) de stérilisation de l'hydrogel obtenu à l'étape (c) est effectuée par exposition de l'hydrogel obtenu à l'étape (c) à des rayonnements ionisants, et plus particulièrement par exposition de l'hydrogel obtenu à l'étape (c) aux rayonnements gamma.

Selon l'invention, l'étape (d) de stérilisation de l'hydrogel obtenu à l'étape (c) est effectuée par exposition de l'hydrogel obtenu à l'étape (c) à des rayonnements gamma compris entre 1 000 Gray et 50 000 Gray, de préférence entre 1 000 Gray et 20 000 Gray.

Selon l'invention, entre l'étape (c) de lyophilisation de l'hydrogel réticulé obtenu à l'étape (b) et l'étape (d) de stérilisation de l'hydrogel obtenu à l'étape (c) sont effectués :
- une étape de compression physique de l'hydrogel lyophilisé,
- et un traitement thermique de l'hydrogel.

De préférence, le traitement thermique est effectué par chauffage dans une gamme de température comprise entre 45 °C et 110 °C.

L'invention a pour objet un produit susceptible d'être obtenu par le procédé décrit ci-dessus.

L'invention a également pour objet un hydrogel susceptible d'être obtenu à partir du produit décrit ci-dessus et d'un liquide aqueux, ledit liquide aqueux comprenant possiblement du sérum et/ou un milieu de culture de cellules.

L'invention a également pour objet un hydrogel dans lequel la concentration de l'acide hyaluronique est comprise entre 1 et 8 mg/mL.

L'invention concerne une utilisation d'un produit ou d'un hydrogel comme décrit ci-dessus dans un milieu pour la culture cellulaire 3D ou dans un milieu pour le transport de cellules, notamment la culture ou le transport de cellules de type adipocytes ou la culture ou le transport de cellules de type hépatocytes.

L'invention a également pour objet un acide hyaluronique modifié par de la galactosamine selon l'étape a').

### 1. Terminologie

Par définition, un hydrogel est un réseau tridimensionnel polymérique dans lequel l'agent gonflant est l'eau (IUPAC PAC, 2007, 79, 1801). Placés dans un environnement aqueux, les hydrogels vont s'hydrater et absorber l'eau présente dans le fluide aqueux. La quantité de fluide aqueux absorbé peut être modulée par le type de macromolécule utilisée. L'hydrogel peut comprendre des additifs tels que des principes actifs.

Le terme « matrice » signifie ici un réseau 3D de macromolécules reliées entre elles par des liaisons covalentes et/ou des liaisons non covalentes.

Le terme « collagènes de type x », x étant compris entre 1 et 28 en chiffres romains selon l'état actuel des connaissances, signifie des collagènes bien connus dans la littérature selon leurs natures chimiques et leurs structures. On se référera à la publication *«* Collagen structure and stability » parue dans l'Annual Review of Biochemistry, vol 78, p929-958 (2009) de M.D. Shoulders et al.

L'acide hyaluronique est abrégé ici « HA ».

### 2. Description des figures

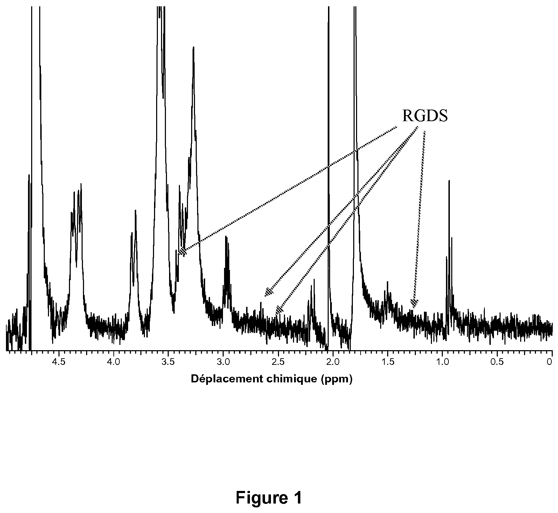

La figure 1 présente un spectre ¹H-RMN de l'acide hyaluronique greffé par du RGDS.

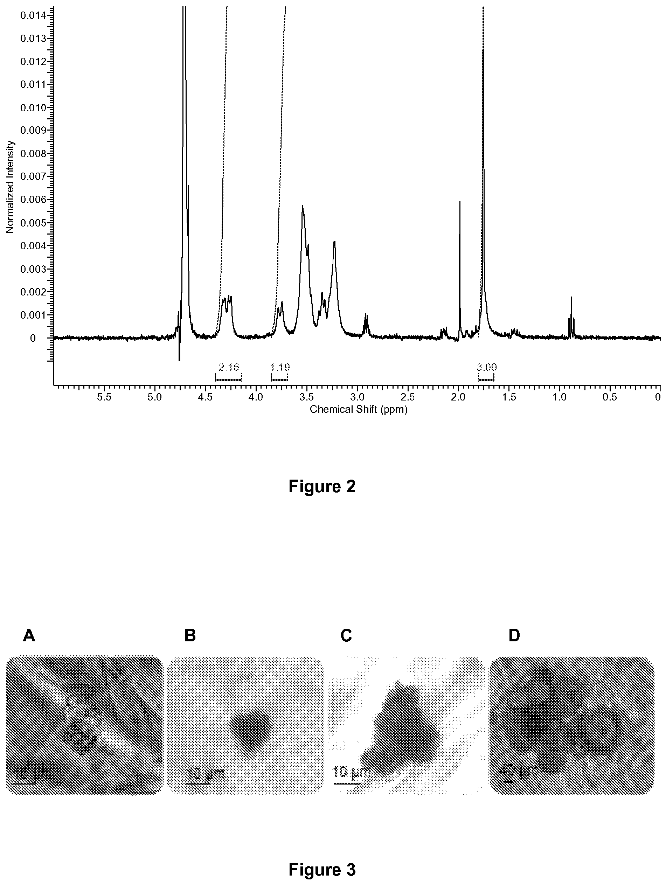

La figure 2 présente un spectre ¹H-RMN de l'acide hyaluronique greffé par de la galactosamine.
La figure 3 présente l'évolution, par microscopie optique inversée, de 40 000 cellules préadipocytes humaines ensemencées sur les hydrogels pour culture cellulaire 3D adipocytes après 1 jour (figure 3 A), 4 jours (figure 3 B), 10 jours (figure 3 C) et 28 jours (figure 3 D) de culture cellulaire.

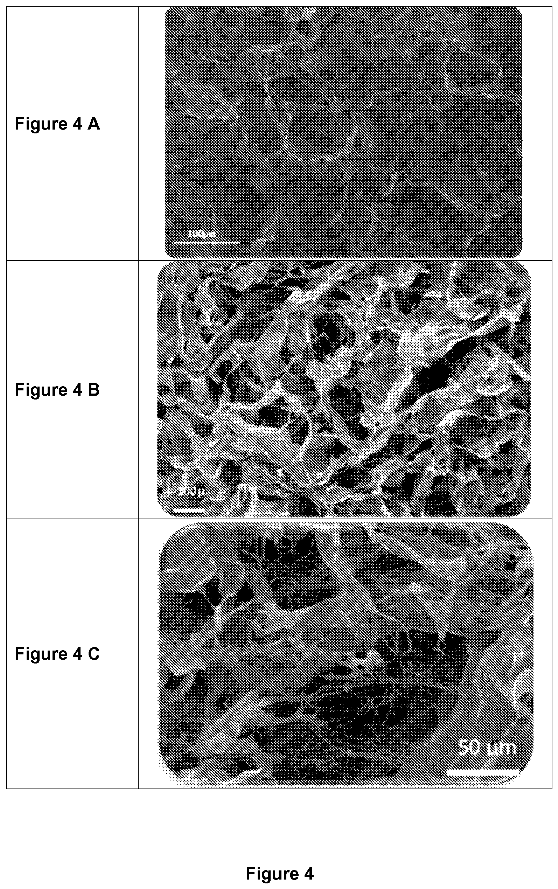

La figure 4 présente des clichés de microscopie électronique à balayage d'hydrogels comprenant de l'acide hyaluronique réticulé seul (cf. figure 4A), de l'acide hyaluronique modifié par du RGDS et de la galactosamine, et reticulé en présence de collagènes de type I et IV (cf. figure 4B), et de l'acide hyaluronique greffé par du RGDS et reticulé en présence de collagènes de type I et VI (cf. figure 4C).

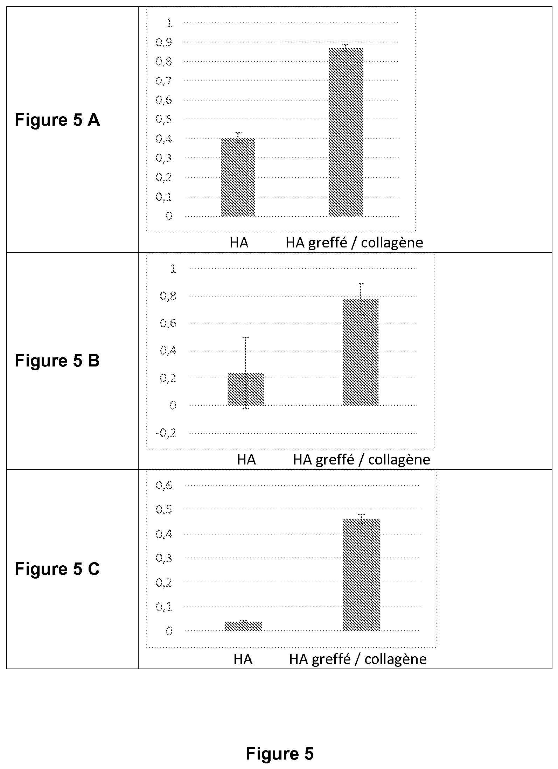

La figure 5 présente les niveaux d'expression de gènes de cellules hépatocytes primaires humaines tels que l'UGT1A1 (cf. figure 5A), le CYP1A1 (cf. figure 5B) et le CYP3A4 (cf. figure 5C) après 7 jours de culture dans des hydrogels d'acide hyaluronique seul (HA) et dans des hydrogels spécialisés selon l'invention comprenant de l'acide hyaluronique greffé par du RGDS et reticulé en présence de collagènes de type I et IV (HA greffé / collagène).

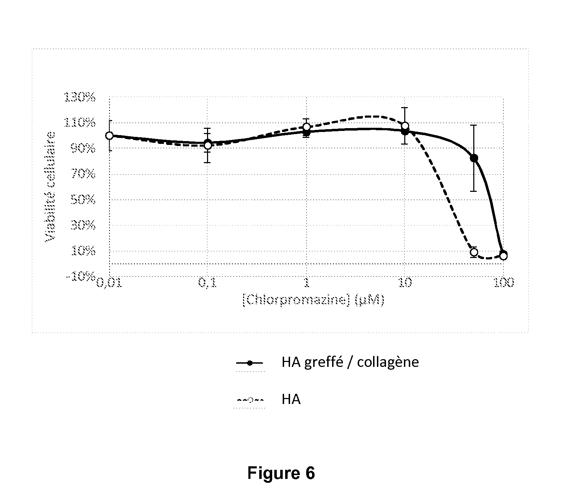

La figure 6 présente la viabilité cellulaire de cellules HepG2. Avant toute mesure de la viabilité cellulaires, les cellules HepG2 sont cultivées pendant 7 jours dans des hydrogels d'acide hyaluronique seul (HA) et dans des hydrogels spécialisés selon l'invention comprenant de l'acide hyaluronique greffé par du RGDS et reticulé en présence de collagènes de type I et IV (HA greffé / collagène), puis traitées à des concentrations de Chlorpromazine comprises entre 0,01 et 100 µM pendant 24h. La viabilité cellulaire est mesurée juste après cette période d'incubation à la Chlorpromazine.

### 3. Description détaillée

La présente invention porte sur un procédé de préparation d'un hydrogel à partir d'acide hyaluronique réticulé en présence de collagène pouvant être utilisé dans de nombreuses applications impliquant la culture cellulaire 3D.

De préférence, l'acide hyaluronique utilisé dans le cadre de la présente invention a une masse moléculaire élevée (> 1x10⁶ Da).

Le procédé général de réalisation des hydrogels selon l'invention est décrit ci-après. Deux variantes du mode de réalisation adaptées à deux types de cultures cellulaires spécifiques sont ensuite présentées. Enfin, l'utilisation de ces différents hydrogels est décrite.

### 3.1. Hydrogel pour culture cellulaire 3D

Afin d'améliorer l'adhésion des cellules au sein de la matrice, des motifs RGDS ont été greffés à l'acide hyaluronique servant de base à la matrice développée. Le motif RGDS permet l'adhésion de plusieurs types de cellules.

### 3.1.1. Modification du HA par greffage du (ARG-GLY-ASP-SER) RGDS

Dans un procédé selon l'invention, la fonctionnalisation du HA par du RGDS peut être réalisée par différents modes de synthèse, et en particulier par voie enzymatique ou par greffage du motif sur un bras espaceur, préalablement greffé sur de l'acide hyaluronique.

Dans un mode de réalisation la fonctionnalisation du HA par du RGDS est réalisée via une réaction de couplage covalente entre une fonction acide de l'acide hyaluronique et une fonction amine du motif RGDS en présence du couple EDCI/NHS (schéma I). Cette réaction de couplage est connue en tant que telle (voir Rowley (1999) « Alginate hydrogels as synthetic extracellular matrix materials », Genes (2004) *«* Effect of substrate mechanics on chondrocyte adhesion to modified alginate surfaces » et Hersel (2003) « RGD modified polymers: biomaterials for stimulated cell adhesion and beyond *»).*

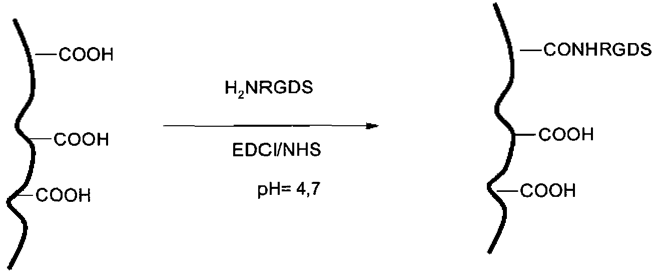

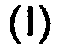

La réaction de couplage entre une fonction acide de l'acide hyaluronique et une fonction amine du motif RGDS est effectuée à température ambiante, via l'ajout stœchiométrique des composés EDCI et NHS dans le milieu réactionnel. Avantageusement on ajoute au milieu réactionnel, une mole d'EDCI/NHS pour une mole de RGDS. En cas d'excès d'EDCI/NHS par rapport au RGDS, des réactions secondaires de réticulation non souhaitées peuvent être observées.

L'EDCI (1-éthyl-3-(3-diméthylaminopropyl)carbodiimide aussi connu sous le sigle EDC ayant pour numéro CAS : 1892-57-5) est un réactif de couplage servant d'activateur du groupement carboxylique et favorise la réaction du groupement carboxylique de l'HA avec le N-hydroxysuccinimide.

Le N-hydroxysuccinimide (NHS ayant pour numéro CAS : 6066-82-6) est un composé organique couramment utilisé en chimie organique comme activateur d'acides carboxyliques et conduisant à l'obtention d'esters activés (ester de succinate).

Cette réaction de couplage entre une fonction acide de l'acide hyaluronique et une fonction amine du motif RGDS en présence du couple EDCI/NHS est une réaction simple effectuée dans un milieu aqueux permettant de conserver l'intégrité de l'acide hyaluronique. L'absence d'utilisation de solvant coûteux (tel que le DMF ou le dichlorométhane) lors de la réaction de couplage permet de réaliser des économies. De plus, ces solvants étant toxiques, ils doivent être éliminés au moyen d'étapes fastidieuses, afin d'éviter la contamination du produit fini par des substances toxiques pour les cellules cultivées. En effet, l'acide hyaluronique modifié doit être exempt de composés toxiques risquant d'entraver son utilisation finale, notamment comme matrice de culture cellulaire 3D.

La réaction est préférentiellement réalisée à un pH compris entre 4 et 5, de préférence à un pH compris entre 4,5 et 4,9 , et encore plus préférentiellement à un pH de 4,75 , de manière à ce que les groupements carboxyliques de l'acide hyaluronique soient sous forme protonée, favorisant ainsi la réaction de couplage entre une fonction acide de l'acide hyaluronique et une fonction amine du composé que l'on souhaite greffer.

Le taux de greffage peut être déterminé par ¹H-RMN. Selon l'invention, le taux de greffage des fonctions acides de l'acide hyaluronique est compris entre 0,5 et 20 % molaire, de préférence 1%. Au-delà d'un taux de greffage de 20% molaire, l'adjonction du motif RGDS induit un encombrement stérique tel, que la réticulation ultérieure de l'acide hyaluronique modifié ne se fait plus de la même manière. Pour un taux de greffage inférieur à 0,5% molaire, l'hydrogel obtenu via la réticulation de l'acide hyaluronique modifié par du RGDS présente un comportement similaire à l'hydrogel obtenu par la simple réticulation de l'acide hyaluronique natif.

### 3.1.2. Réticulation des HA modifiés en présence de collagènes I par une solution aqueuse et/ou alcoolique d'un agent de réticulation

Dans un échafaudage, les molécules d'acide hyaluronique sont réticulées par des liaisons chimiques covalentes appropriées, i.e. via des ponts de type aldéhyde, des ponts disulfures. De nombreuses méthodes de réticulation d'acide hyaluronique pour la préparation d'hydrogels sont connues de l'homme du métier, et notamment décrites dans la publication de Prestwich et al, J. Control.Release, 1998, 53: 93-103.

La réticulation des acides hyaluroniques modifiés par du RGDS est effectuée via l'emploi d'une solution aqueuse et/ou alcoolique d'un agent de réticulation tel que l'ADH, et ce, en présence de collagènes de type I (cf. schéma II). La présence d'acide hyaluronique et de collagène de type I au sein de l'hydrogel permet de mimer la matrice extracellulaire et ainsi favoriser la culture cellulaire.

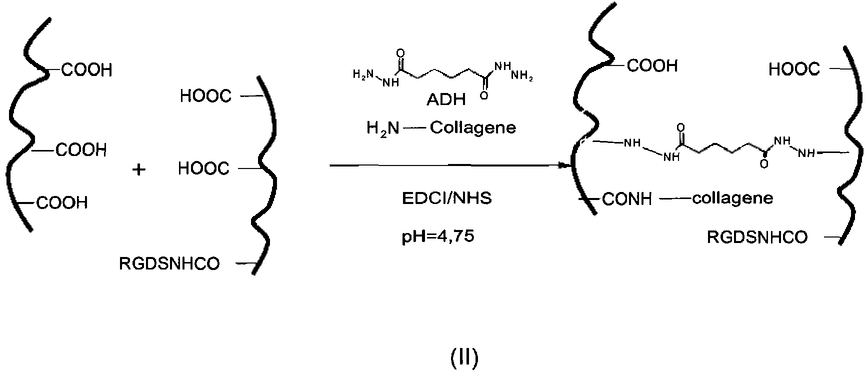

En tant qu'agent de réticulation, on peut utiliser une solution aqueuse d'acide adipique dihydrazide (ADH) contenant de l'EDCI ou contenant le couple EDCI / NHS. L'utilisation de l'ADH comme agent réticulant permet de s'affranchir de solvants et/ou d'agents de réticulation bien souvent toxiques et permet d'effectuer cette réaction de réticulation dans des conditions douces, à savoir à température ambiante et dans un milieu réactionnel dont la gamme de pH est comprise entre 4 et 7. Comme précédemment, l'EDCI et le NHS servent d'activateurs d'acides carboxyliques, groupements présents sur la chaîne d'acide hyaluronique.

La solution aqueuse et/ou alcoolique d'un agent de réticulation peut comprendre un solvant alcoolique tel que l'éthanol, le propanol, l'isopropanol et le butanol. L'utilisation d'une solution aqueuse et/ou alcoolique présente un avantage significatif car cela permet de s'affranchir de solvants très toxiques (DMSO, DMF Dichlorométhane) et facilite la purification ultérieure du milieu réactionnel, qui peut être réalisée notamment par dialyse.

D'autres agents de réticulation peuvent être employés. Les composés dihydrazides tels que l'acide sebacique dihydrazide, le dodecanediohydrazide, l'acide isophtalique dihydrazide, l'acide succinique dihydrazide ou encore les composés diamines possédant deux fonctions amines primaires terminales (H₂N-R-NH₂ où R représente n'importe quel groupement entre les 2 fonctions amines terminales) peuvent être utilisés. Les agents de réticulations employés dans le procédé selon l'invention doivent posséder une bonne solubilité dans le milieu de réticulation afin de favoriser la réaction de réticulation. Dans une solution aqueuse et/ou alcoolique d'un agent de réticulation, la solubilité des composés diamines ou dihydrazides employés comme agent de réticulation est fonction de la longueur de la chaine alkyle (R) présente entre les deux fonctions amines primaires terminales des composés usités. On observe que la solubilité des composés diamines ou dihydrazides est inversement proportionnelle à la longueur de la chaine alkyle (R) présente entre les deux fonctions amines primaires terminales des composés usités.

Afin d'éviter toute dénaturation de l'hydrogel lors d'une étape ultérieure de chauffage à 100°C, les agents de réticulation choisis et employés dans l'élaboration de l'hydrogel doivent résister à des températures de l'ordre de 100°C.

La réaction de réticulation est préférentiellement réalisée à un pH compris entre 4 et 5, de préférence à un pH compris entre 4,5 et 4,9 , et encore plus préférentiellement à un pH de 4,75 , de manière à ce que les groupements carboxyliques de l'acide hyaluronique soient sous forme protonée, favorisant ainsi la réaction de couplage entre une fonction acide de l'acide hyaluronique et une fonction amine du composé que l'on souhaite greffer.

La concentration du HA dans le gel est avantageusement comprise entre 1 et 8 mg/ml ; une valeur de 4 mg/ml donne de très bons résultats. Une concentration du HA inférieure à 8 mg/ml peut être obtenue par dilution de l'hydrogel.

La co-réticulation du collagène de type I et de l'acide hyaluronique en présence d'ADH est effectuée à des ratios collagène de type I / acide hyaluronique inférieurs ou égaux à 15% molaire. Au-delà d'un ratio molaire collagène de type I / acide hyaluronique égal à 15%, le taux de réticulation n'est pas suffisant pour permettre l'obtention d'un hydrogel. Pour des ratios molaires collagène de type I / acide hyaluronique inférieurs à 0,1 %, préférentiellement inférieurs à 0.2 % et encore plus préférentiellement inférieures à 0.25 %, l'hydrogel obtenu se comporte comme un hydrogel d'acide hyaluronique pur.

Le mélange réactionnel est ensuite laissé gélifier pendant environ 12 heures à température ambiante. Dans un mode de réalisation avantageux, la gélification est effectuée la première heure sous agitation douce puis le gel est laissé sans agitation, pendant environ 11 heures, à température ambiante, afin que le gel puisse se stabiliser.

Les hydrogels d'acide hyaluronique réticulés obtenus sont ensuite purifiés par séparation membranaire. Cette séparation membranaire peut être effectuée de manière connue, par toute technique appropriée telle que la diafiltration via l'emploi de membranes présentant une valeur de MWCO (« molecular weight cut-off », valeur de coupure de la masse moléculaire) comprise entre 10 kDa et 30 kDa, par exemple entre 12 kDa et 14 kDa en mode boudin.

L'hydrogel peut être équilibré dans une solution aqueuse saline, dans un mélange eau / éthanol (par exemple avec le rapport volumique eau 3/4, éthanol 1/4) ou encore dans une solution d'eau milliQ afin d'éliminer les composés n'ayant pas réagi, tels que l'agent de réticulation.

Les hydrogels d'acide hyaluronique réticulés purifiés sont ensuite coulés dans des plaques, congelés (par exemple à -20°C) et lyophilisés afin d'éliminer le solvant, i.e. la solution aqueuse ou la solution hydroalcoolique contenue dans l'hydrogel.

L'hydrogel précédemment lyophilisé va ensuite être comprimé dans une étape de compression mécanique. Cette étape de compression mécanique de l'hydrogel lyophilisé peut être réalisée par tout moyen de compression. Cette étape de compression permet, d'une part, de compacter le matériau et de rapprocher les chaînes polymériques les unes des autres, et d'autre part, d'accroitre la tenue mécanique ultérieure de l'hydrogel, notamment lors de son utilisation en culture cellulaire.

L'hydrogel se présente alors sous la forme d'une pastille lyophilisée.

L'hydrogel précédemment lyophilisé et comprimé va ensuite faire l'objet d'un traitement thermique, i.e. une étape de chauffage. L'étape de chauffage peut être réalisée via tout moyen de chauffage. Cette étape de chauffage permet de renforcer l'hydrogel, tout en conservant l'intégrité de la structure de l'hydrogel. Les hydrogels sont préférentiellement chauffés dans une gamme de température comprise entre 45 °C et 110 °C en utilisant un bain d'huile ou tout autre moyen de chauffage. Cette étape de chauffage de l'hydrogel confère une certaine rigidité à la matrice via notamment la formation de liaisons hydrogènes entre les chaînes polymériques de l'hydrogel. Un chauffage de l'hydrogel à une température supérieure à 110 °C dénature la structure de l'hydrogel. Un chauffage de l'hydrogel a une température inférieure à 45 °C n'induit pas une rigidité suffisante de la matrice.

L'hydrogel est ensuite réhydraté dans un milieu aqueux contenant de l'acide hyaluronique, transféré dans la plaque de destination finale, congelé puis lyophilisé dans un lyophilisateur.

La présence d'acide hyaluronique dans le milieu de réhydratation confère ultérieurement à l'hydrogel ainsi lyophilisée de meilleures propriétés d'adhésion à la surface de la plaque de destination finale. La concentration de l'acide hyaluronique dans le milieu aqueux est comprise entre 0,5 g/L et 4 g/L. La teneur en acide hyaluronique dans le milieu de réhydratation est, de préférence, de 2 g par litre d'eau.

Après la congélation et la lyophilisation de l'hydrogel, ce dernier se présente alors sous la forme d'un hydrogel lyophilisé.

La stérilisation de l'hydrogel lyophilisé ainsi obtenu est effectuée par exposition des produits à traiter, i.e. hydrogels, à des rayonnements fortement ionisants, notamment aux rayonnements gamma. Avantageusement, on administre une dose de rayonnement comprise entre 1 000 Gray et 50 000 Gray, de préférence entre 1 000 Gray et 20 000 Gray. Cette étape est une méthode de décontamination qui, avantageusement, ne dégrade pas l'hydrogel.

Les hydrogels sont ensuite stockés sous conditions stériles, à l'abri de l'air, de l'humidité et de la lumière avant toute réhydratation.

Le produit final se présente sous la forme d'une pastille. Il peut être transformé en hydrogel en l'hydratant dans une quantité voulue d'un milieu aqueux. Le milieu aqueux peut comprendre des additifs, tels que des facteurs de croissance et/ou des principes pharmaceutiques actifs (tels que des antibiotiques), ou encore du sérum. Afin d'utiliser ce produit comme hydrogel utilisable comme matrice cellulaire 3D, les hydrogels peuvent être directement ensemencés avec les cellules d'intérêt dans un volume dépendant du type cellulaire et du protocole développé (i.e. 50µL avec 50000 cellules). Suivant le type cellulaire, le milieu de culture et le protocole développé, le milieu peut être renouvelé toutes les 48 heures pour favoriser la croissance des cellules. La matrice cellulaire 3D peut ensuite être utilisée plusieurs semaines après la mise en culture cellulaire, i.e. l'ensemencement de l'hydrogel de manière à ce que la croissance des cellules d'intérêt soit suffisante.

### 3.2. Hydrogel pour culture cellulaire 3D d'hépatocytes

Pour la culture de cellules hépatocytaires, la matrice extracellulaire du foie peut être mimée par un hydrogel réalisé selon l'invention. Le foie comprend de nombreuses macromolécules dont de l'acide hyaluronique et différents types de collagènes comme le souligne la publication « Liver Fibrosis: Perspectives in Pathobiochemical Research and Clinical Outlook » de A. M. Gressner (1991). Plusieurs études montrent que le collagène type I et type IV sont les deux collagènes caractéristiques d'un changement d'état du foie *(*M. Kwiecinski 2011 : « Hepatocyte Growth Factor (HGF) Inhibits Collagen I and IV Synthesis in Hepatic Stellate Cells by miRNA-29 Induction », PloS ONE, 6(9) (2011) e24568 p1-13). Le collagène de type I est caractérisé par une structure de type fibrille fibrillaire (Matthew D. Shoulders 2009 : « Collagen structure and stability *».* Le collagène de type IV se présente sous forme de feuillets (Hudson BG 1993: « Type IV collagen: structure, gene organization, and role in human diseases. Molecular basis of Goodpasture and AIport syndromes and diffuse leiomyomatosis*").*

De manière à pouvoir mimer au mieux l'environnement des cellules hépatocytes, l'hydrogel selon la variante « culture d'hépatocytes » de l'invention, comporte avantageusement de l'acide hyaluronique et des collagènes de type I et IV.

Afin d'améliorer l'adhésion des cellules au sein de la matrice, des motifs ont été greffés à l'acide hyaluronique servant de base à la matrice développée. Ces motifs peuvent être des motifs peptidiques de type RGDS ou encore d'autres motifs tels que des motifs galactosamine (H2NGal). Ces molécules sont greffées sur le support HA par des liaisons chimiques covalentes.

Ainsi, dans un mode de réalisation de l'invention, on fonctionnalise l'acide hyaluronique par un motif RGDS comme présenté précédemment. Le taux de greffage des fonctions acides de l'acide hyaluronique est de 1%.

### 3.2.1. Modification du HA par greffage du galactosamine

La fonctionnalisation du HA par un motif galactosamine est réalisée via une réaction de couplage entre une fonction acide de l'acide hyaluronique et une fonction amine du motif galactosamine en présence du couple EDCI/NHS selon le même protocole de synthèse que celui employé pour réaliser de l'acide hyaluronique modifié par du RGDS.

Le taux de greffage peut être déterminé par ¹H-RMN. Selon l'invention, le taux de greffage des fonctions acides de l'acide hyaluronique est compris entre 0,5 et 25 % molaire, de préférence 12%. Au-delà d'un taux de greffage de 25% molaire, l'adjonction du motif galactosamine induit un tel encombrement stérique que la réticulation ultérieure de l'acide hyaluronique modifié ne se fait plus de la même manière. Pour un taux de greffage inférieur à 0,5% molaire, l'hydrogel obtenu, via la réticulation de l'acide hyaluronique modifié par de la galactosamine, présente un comportement similaire à l'hydrogel obtenu par la simple réticulation de l'acide hyaluronique natif.

### 3.2.2. Réticulation des HA modifiés en présence de collagènes I et IV par une solution aqueuse et/ou alcoolique d'un agent de réticulation

La réticulation des acides hyaluroniques modifiés par de la galactosamine et du RGDS est effectuée via l'emploi d'une solution aqueuse et/ou alcoolique d'un agent de réticulation, en présence de collagènes de type I et IV. La proportion des collagènes de type I et IV dans le milieu réactionnel est choisie de manière à favoriser l'adhésion cellulaire. La proportion de collagène de type I dans le milieu réactionnel est avantageusement comprise entre 0,1 % et 0,4 % molaire; une valeur de 0,3 % donne de très bons résultats. La proportion de collagène de type IV dans le milieu réactionnel est avantageusement comprise entre 0,05 % et 0,15 % molaire; une valeur de 0,07 % donne de très bons résultats. Ainsi, avoir 0,3% molaire de collagène I et 0,07% molaire de collagène IV dans le milieu réactionnel confère ultérieurement de bonnes propriétés à l'hydrogel final.

L'agent de réticulation employé peut être une solution aqueuse d'acide adipique dihydrazide (ADH) contenant de l'EDCI, ou de l'EDCI et du NHS. L'utilisation de l'ADH comme agent réticulant permet de s'affranchir de solvants bien souvent toxiques et d'effectuer cette réaction de réticulation dans des conditions douces. Comme précédemment, l'EDCI et le NHS servent d'activateurs d'acides carboxyliques, groupements présents sur la chaîne d'acide hyaluronique. La solution aqueuse et/ou alcoolique d'un agent de réticulation peut comprendre un solvant alcoolique tel que l'éthanol, le propanol, l'isopropanol et le butanol. L'utilisation d'une solution aqueuse et/ou alcoolique présente un avantage significatif car cela permet de s'affranchir de solvants très toxiques (DMSO, DMF Dichlorométhane) et facilite la purification ultérieure du milieu réactionnel, qui peut être réalisée notamment par dialyse.

D'autres agents de réticulation peuvent être employés. Les composés dihydrazides tels que l'acide sebacique dihydrazide, le dodecanediohydrazide, l'acide isophtalique dihydrazide, l'acide succinique dihydrazide ou encore les composés diamines possédant deux fonctions amines primaires terminales (H₂N-R-NH₂ où R représente n'importe quel groupement entre les 2 fonctions amines terminales) peuvent être utilisés. Les agents de réticulations employés dans le procédé selon l'invention doivent posséder une bonne solubilité dans le milieu de réticulation, afin de favoriser la réaction de réticulation. Dans une solution aqueuse et/ou alcoolique d'un agent de réticulation, la solubilité des composés diamines ou dihydrazides employés comme agent de réticulation est fonction de la longueur de la chaine alkyle (R) présente entre les deux fonctions amines primaires terminales des composés usités. On observe que la solubilité des composés diamines ou dihydrazides est inversement proportionnelle à la longueur de la chaine alkyle (R) présente entre les deux fonctions amines primaires terminales des composés usités.

Afin d'éviter toute dénaturation de l'hydrogel lors d'une étape ultérieure de stérilisation par chauffage à 100°C, les agents de réticulation choisis et employés dans l'élaboration de l'hydrogel doivent résister à des températures de l'ordre de 100°C.

La réaction de réticulation est préférentiellement réalisée à un pH compris entre 4 et 5, de préférence à un pH compris entre 4,5 et 4,9 , et encore plus préférentiellement à un pH de 4,75 , de manière à ce que les groupements carboxyliques de l'acide hyaluronique soient sous forme protonée, favorisant ainsi la réaction de couplage entre une fonction acide de l'acide hyaluronique et une fonction amine du composé que l'on souhaite greffer.

La concentration du HA dans le gel est avantageusement comprise entre 1 et 8 mg/ml ; une valeur de 4 mg/ml donne de très bons résultats. Une concentration du HA inférieure à 8 mg/ml peut être obtenue par dilution de l'hydrogel.

La co-réticulation du collagène de type I et IV et de l'acide hyaluronique en présence d'ADH est effectuée à des ratios collagène de type I et IV / acide hyaluronique inférieurs ou égaux à 10 % massique, i.e. 15 % molaire. Au-delà d'un ratio molaire collagène de type I et IV / acide hyaluronique égal à 15 %, le taux de réticulation n'est pas suffisant pour permettre l'obtention d'un hydrogel. Pour des ratios molaires collagène de type I et IV / acide hyaluronique inférieurs à 0,1 %, préférentiellement inférieurs à 0.2 % et encore plus préférentiellement inférieures à 0,25 %, l'hydrogel obtenu se comporte comme un hydrogel d'acide hyaluronique pur.

Le mélange réactionnel est ensuite laissé gélifier pendant environ 12 heures à température ambiante. Dans un mode de réalisation préféré, la gélification est effectuée la première heure sous agitation douce, puis le gel est laissé sans agitation, pendant environ 11 heures, à température ambiante, afin que le gel puisse se stabiliser.

Les hydrogels d'acide hyaluronique réticulés obtenus sont ensuite purifiés par séparation membranaire. Cette séparation membranaire peut être effectuée de manière connue, par toute technique appropriée telle que la diafiltration via l'emploi de membranes présentant une valeur de MWCO (« molecular weight cut-off ») compris entre 10 kDa et 30 kDa, par exemple entre 12 kDa et 14 kDa en mode boudin.

L'hydrogel peut être équilibré dans une solution aqueuse saline, dans un mélange eau / éthanol (par exemple avec le rapport volumique eau 3/4, éthanol 1/4) ou encore dans une solution d'eau milliQ afin d'éliminer les composés n'ayant pas réagi, tels que l'agent de réticulation.

Les hydrogels d'acide hyaluronique réticulés purifiés sont ensuite coulés dans des plaques, congelés (par exemple à -20°C) et lyophilisés afin d'éliminer le solvant, i.e. la solution aqueuse ou la solution hydroalcoolique contenue dans l'hydrogel.

L'hydrogel précédemment lyophilisé va ensuite être comprimé dans une étape de compression mécanique. Cette étape de de compression mécanique de l'hydrogel lyophilisé peut être réalisée par tout moyen de compression. Cette étape de compression permet d'une part de compacter le matériau, de rapprocher les chaînes polymériques les unes des autres, et d'autre part d'accroitre la tenue mécanique ultérieure de l'hydrogel, notamment lors de son utilisation en culture cellulaire.

L'hydrogel se présente alors sous la forme d'une pastille lyophilisée.

L'hydrogel précédemment lyophilisé et comprimé va ensuite faire l'objet d'un traitement thermique, i.e. une étape de chauffage. L'étape de chauffage peut être réalisée via tout moyen de chauffage. Cette étape de chauffage permet de renforcer l'hydrogel, tout en conservant l'intégrité de la structure de l'hydrogel. Les hydrogels sont préférentiellement chauffés dans une gamme de température comprise entre 45 °C et 110 °C en utilisant un bain d'huile ou tout autre moyen de chauffage. Cette étape de chauffage de l'hydrogel confère une certaine rigidité à la matrice via notamment la formation de liaisons hydrogènes entre les chaînes polymériques de l'hydrogel. Un chauffage de l'hydrogel à une température supérieure à 110 °C dénature la structure de l'hydrogel. Un chauffage de l'hydrogel a une température inférieure à 45 °C n'induit pas une rigidité suffisante de la matrice.

L'hydrogel est ensuite réhydraté dans un milieu aqueux contenant de l'acide hyaluronique, transféré dans la plaque de destination finale, congelé puis lyophilisé dans un lyophilisateur.

La présence d'acide hyaluronique dans le milieu de réhydratation confère ultérieurement à l'hydrogel ainsi lyophilisée de meilleures propriétés d'adhésion à la surface de la plaque de destination finale. La concentration de l'acide hyaluronique dans le milieu aqueux est comprise entre 0,5 g/L et 4 g/L. La teneur en acide hyaluronique dans le milieu de réhydratation est, de préférence, de 2 g par litre d'eau.

Après la congélation et la lyophilisation de l'hydrogel, ce dernier se présente alors sous la forme d'un hydrogel lyophilisé.

La stérilisation de l'hydrogel lyophilisé ainsi obtenu est effectuée par exposition des produits à traiter, i.e. hydrogels, à des rayonnements fortement ionisants, notamment aux rayonnements gamma. Avantageusement, on administre une dose de rayonnement comprise entre 1000 Gray et 50 000 Gray, de préférence entre 1 000 Gray et 20 000 Gray. Cette étape est une méthode de décontamination qui, avantageusement, ne dégrade pas l'hydrogel.

Les hydrogels sont ensuite stockés sous conditions stériles, à l'abri de l'air, de l'humidité et de la lumière avant toute réhydratation.

Le produit final se présente sous la forme d'une pastille. Il peut être transformé en hydrogel en l'hydratant dans une quantité voulue d'un milieu aqueux. Le milieu aqueux peut comprendre des additifs, tels que des facteurs de croissance et/ou des principes pharmaceutiques actifs (tels que des antibiotiques), ou encore du sérum. Afin d'utiliser ce produit comme hydrogel utilisable comme matrice cellulaire 3D, les hydrogels peuvent être directement ensemencés avec les cellules d'intérêt dans un volume dépendant du type cellulaire et du protocole développé (i.e. 50µL avec 50000 cellules). Suivant le type cellulaire, le milieu de culture et le protocole développé, le milieu peut être renouvelé toutes les 48 heures pour favoriser la croissance des cellules. La matrice cellulaire 3D peut ensuite être utilisée plusieurs semaines après la mise en culture cellulaire, i.e. l'ensemencement de l'hydrogel de manière à ce que la croissance cellulaire des cellules d'intérêt soit suffisante.

### 3.3. Hydrogel pour culture cellulaire 3D d'adipocytes

Ainsi, dans un autre mode de réalisation de l'invention, on fonctionnalise l'acide hyaluronique par un motif RGDS comme présenté précédemment. Le taux de greffage des fonctions acides de l'acide hyaluronique est avantageusement de l'ordre de 1%.

### 3.3.1. Réticulation des HA modifiés en présence de collagènes I et VI par une solution aqueuse et/ou alcoolique d'un agent de réticulation

La réticulation des acides hyaluroniques modifiés par du RGDS est effectuée via l'emploi d'une solution aqueuse et/ou alcoolique d'un agent de réticulation, et ce, en présence de collagènes de type I et VI, principaux constituants de l'environnement des cellules adipocytes.

L'agent de réticulation employé peut être une solution aqueuse d'acide adipique dihydrazide (ADH) contenant de l'EDCI ou contenant de l'EDCI et du NHS. Le fait d'utiliser de l'ADH comme agent réticulant, permet de s'affranchir de l'utilisation de solvants bien souvent toxiques et d'effectuer cette réaction de réticulation dans des conditions douces. Comme précédemment, l'EDCI et le NHS servent d'activateurs d'acides carboxyliques, groupements présents sur la chaîne d'acide hyaluronique.

La solution aqueuse et/ou alcoolique d'un agent de réticulation peut comprendre un solvant alcoolique tel que l'éthanol, le propanol, l'isopropanol et le butanol. L'utilisation d'une solution aqueuse et/ou alcoolique présente un avantage significatif car cela permet de s'affranchir de solvants très toxiques (DMSO, DMF Dichlorométhane) et facilite la purification ultérieure du milieu réactionnel, qui peut être réalisée notamment par dialyse.

D'autres agents de réticulation peuvent être employés. Les composés dihydrazides tels que l'acide sebacique dihydrazide, le dodecanediohydrazide, l'acide isophtalique dihydrazide, l'acide succinique dihydrazide ou encore les composés diamines possédant deux fonctions amines primaires terminales (H₂N-R-NH₂ où R représente n'importe quel groupement entre les 2 fonctions amines terminales) peuvent être usités. Les agents de réticulations employés dans le procédé selon l'invention doivent posséder une bonne solubilité dans le milieu de réticulation, et ce, afin de favoriser la réaction de réticulation. Dans une solution aqueuse et/ou alcoolique d'un agent de réticulation, la solubilité des composés diamines ou dihydrazides employés comme agent de réticulation est fonction de la longueur de la chaine alkyle (R) présente entre les deux fonctions amines primaires terminales des composés usités. On constate que la solubilité des composés diamines ou dihydrazides inversement proportionnelle à la longueur de la chaine alkyle (R) présente entre les deux fonctions amines primaires terminales des composés usités.

De plus, les agents de réticulation choisis et employés dans l'élaboration de l'hydrogel doivent résister à des températures de l'ordre de 100°C, et ce afin d'éviter toute dénaturation de l'hydrogel lors d'une étape ultérieure de stérilisation par chauffage à 100°C.

La réaction de réticulation est préférentiellement réalisée à un pH compris entre 4 et 5, préférentiellement compris entre 4,5 et 4,9 , et encore plus préférentiellement à un pH de 4,75 , de manière à ce que les groupements carboxyliques de l'acide hyaluronique soient sous forme protonée, favorisant ainsi la réaction de couplage entre une fonction acide de l'acide hyaluronique et une fonction amine du composé que l'on souhaite greffer.

La concentration du HA dans le gel est avantageusement comprise entre 1 et 8 mg/ml ; une valeur de 4 mg/ml donne de très bons résultats.

La co-réticulation du collagène de type I et VI et de l'acide hyaluronique en présence d'ADH est effectuée à des ratios collagène de type I et VI / acide hyaluronique inférieurs ou égaux à 10 % massique, i.e. 15 % molaire. Au-delà d'un ratio molaire collagène de type I et VI / acide hyaluronique égal à 15 %, le taux de réticulation n'est pas suffisant pour permettre l'obtention d'un hydrogel. Pour des ratios molaires collagène de type I et VI / acide hyaluronique inférieurs à 0,1 %, préférentiellement inférieurs à 0.2 % et encore plus préférentiellement inférieures à 0,25 %, l'hydrogel obtenu se comporte comme un hydrogel d'acide hyaluronique pur.

Le mélange réactionnel est ensuite laissé gélifier pendant environ 12 heures à température ambiante. La gélification est effectuée la première heure sous agitation douce puis le gel est laissé sans agitation, pendant environ 11 heures, à température ambiante, afin que le gel puisse se stabiliser.

Les hydrogels d'acide hyaluronique réticulés obtenus sont ensuite purifiés par séparation membranaire. Cette séparation membranaire peut être effectuée de manière connue, par exemple par toute technique appropriée telle que la diafiltration via l'emploi de membranes présentant une valeur de MWCO (« molecular weight cut-off ») compris entre 10 kDa et 30 kDa, par exemple entre 12 kDa et 14 kDa en mode boudin.

L'hydrogel peut être équilibré dans une solution aqueuse saline, dans un mélange eau / éthanol (par exemple avec le rapport volumique eau 3/4, éthanol 1/4) ou encore dans une solution d'eau milliQ afin d'éliminer les composés n'ayant pas réagi, tels que l'agent de réticulation.

Les hydrogels d'acide hyaluronique réticulés purifiés sont ensuite coulés dans des plaques, congelés (par exemple à -20°C) et lyophilisés afin d'éliminer le solvant, i.e. la solution aqueuse ou la solution hydroalcoolique contenue dans l'hydrogel.

L'hydrogel précédemment lyophilisé va ensuite être comprimé dans une étape de compression mécanique. Cette étape de de compression mécanique de l'hydrogel lyophilisé peut être réalisée par tout moyen de compression. Cette étape de compression permet d'une part de compacter le matériau, de rapprocher les chaînes polymériques les unes des autres, et d'autre part d'accroitre la tenue mécanique ultérieure de l'hydrogel, notamment lors de son utilisation en culture cellulaire.

L'hydrogel se présente alors sous la forme d'une pastille lyophilisée.

L'hydrogel précédemment lyophilisé et comprimé va ensuite faire l'objet d'un traitement thermique, i.e. une étape de chauffage. L'étape de chauffage peut être réalisée via tout moyen de chauffage. Cette étape de chauffage permet de renforcer l'hydrogel, tout en conservant l'intégrité de la structure de l'hydrogel. Les hydrogels sont préférentiellement chauffés dans une gamme de température comprise entre 45 °C et 110 °C en utilisant un bain d'huile ou tout autre moyen de chauffage. Cette étape de chauffage de l'hydrogel confère une certaine rigidité à la matrice via notamment la formation de liaisons hydrogènes entre les chaînes polymériques de l'hydrogel. Un chauffage de l'hydrogel à une température supérieure à 110 °C dénature la structure de l'hydrogel. Un chauffage de l'hydrogel a une température inférieure à 45 °C n'induit pas une rigidité suffisante de la matrice.

L'hydrogel est ensuite réhydraté dans un milieu aqueux contenant de l'acide hyaluronique, transféré dans la plaque de destination finale, congelé puis lyophilisé dans un lyophilisateur.

La présence d'acide hyaluronique dans le milieu de réhydratation confère ultérieurement à l'hydrogel ainsi lyophilisée de meilleures propriétés d'adhésion à la surface de la plaque de destination finale. La concentration de l'acide hyaluronique dans le milieu aqueux est comprise entre 0,5 g/L et 4 g/L. La teneur en acide hyaluronique dans le milieu de réhydratation est, de préférence, de 2 g par litre d'eau.

Après la congélation et la lyophilisation de l'hydrogel, ce dernier se présente alors sous la forme d'un hydrogel lyophilisé.

La stérilisation de l'hydrogel lyophilisé ainsi obtenu est effectuée par exposition des produits à traiter, i.e. hydrogels, à des rayonnements fortement ionisants, notamment aux rayonnements gamma. Avantageusement, on administre une dose de rayonnement comprise entre 1 000 Gray et 50 000 Gray, de préférence entre 1 000 Gray et 20 000 Gray. Cette étape est une méthode de décontamination qui, avantageusement, ne dégrade pas l'hydrogel.

Les hydrogels sont ensuite stockés sous conditions stériles, à l'abri de l'air, de l'humidité et de la lumière avant toute réhydratation.

Le produit final se présente sous la forme d'une pastille. Il peut être transformé en hydrogel en l'hydratant dans une quantité voulue d'un milieu aqueux. Le milieu aqueux peut comprendre des additifs, tels que des facteurs de croissance et/ou des principes pharmaceutiques actifs (tels que des antibiotiques), ou encore du sérum. Afin d'utiliser ce produit comme hydrogel utilisable comme matrice cellulaire 3D, les hydrogels peuvent être directement ensemencés avec les cellules d'intérêt dans un volume dépendant du type cellulaire et du protocole développé (i.e. 50µL avec 50000 cellules). Suivant le type cellulaire, le milieu de culture et le protocole développé, le milieu peut être renouvelé toutes les 48 heures pour favoriser la croissance des cellules. La matrice cellulaire 3D peut ensuite être utilisée plusieurs semaines après la mise en culture cellulaire, i.e. l'ensemencement de l'hydrogel de manière à ce que la croissance cellulaire des cellules d'intérêt soit suffisante.

### 4. Exemples

L'invention est illustrée ci-dessous par des exemples qui cependant ne limitent pas l'invention. Ces exemples portent sur l'élaboration d'hydrogels et leurs utilisations comme milieu ou support de cultures cellulaires tridimensionnelles.

### 4.1. Exemple 1 : Hydrogel pour culture cellulaire 3D d'adipocytes

La matrice hydrogel spécialisée culture cellulaire adipocyte a été préparée en deux étapes.

### 4.1.1. Modification du HA par greffage du (ARG-GLY-ASP-SER) RGDS

Dans certains modes de réalisation préférés, les acides hyaluroniques utilisés ont un poids moléculaire élevé (> 1x10⁶ Da).

Le schéma réactionnel du greffage du tetrapeptide RGDS sur l'acide hyaluronique est présenté ci-après (Schéma I).

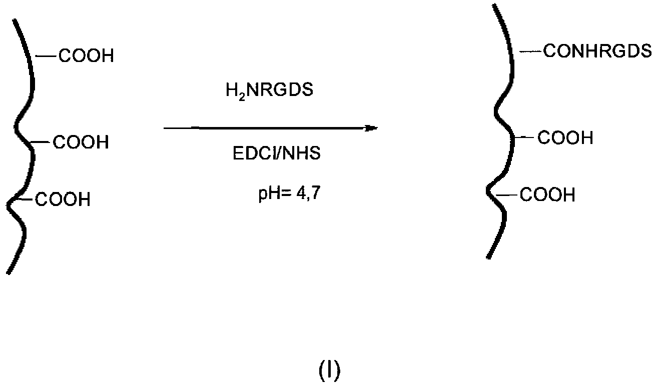

400 mg d'acide hyaluronique et 17 mg de tetrapeptide RGDS ont été ajoutés et dissous dans 0,1 litre d'eau.

5 mg de N-hydroxysuccinimide (NHS) et 6 mg d'ethyl(dimethylaminopropyl) carbodiimide (EDCI ou 1-éthyl-3-(3-diméthylaminopropyl)carbodiimide ; fourni par TCI Europe) ont été ajoutées sans purification préalable à température ambiante dans la solution aqueuse d'acide hyaluronique contenant le tetrapeptide RGDS précédemment préparée. Les ratios NHS/ acide hyaluronique et d'EDCl/acide hyaluronique ont été ajustés afin d'obtenir des acides hyaluroniques greffés par du RGDS optimisés pour l'adhésion de cellules et pour la culture cellulaire.

Le pH, de la solution contenant le tetrapeptide RGDS et l'acide hyaluronique, a été fixé à 4,75 par l'ajout d'HCl 0,1 M. Le mélange a ensuite été agité pendant 12 heures à température ambiante afin d'obtenir de l'acide hyaluronique greffé par du RGDS.

La purification du HA greffé par du (ARG-GLY-ASP-SER) RGDS a été effectuée par filtration tangentielle.

L'acide hyaluronique greffé par du RGDS a ensuite été placé dans un récipient en plastique, congelé puis lyophilisé dans un lyophilisateur (Alpha 1-2 - performances: 2 kg de glace par 24 heures, T = -55 ° C) durant 24 heures.

Le taux de greffage de motif RGDS a été déterminé par ¹H-RMN (cf. figure 1) et est de l'ordre de 1%.

### 4.1.2. Réticulation des HA modifiés en présence de collagènes I et VI par l'ADH

Les hydrogels d'acides hyaluroniques modifiés ont été préparés comme le montre le schéma réactionnel suivant (II), à partir de l'acide hyaluronique greffé par du RGDS, obtenu à l'étape 1, du collagène de type I, du collagène de type VI, de l'acide adipique dihydrazide (ADH, fourni par TCI Europe) utilisé comme agent de réticulation et à partir d'EDCI (i.e. I-éthyl-3 [3- (diméthylamino) propyl] carbodiimide, fourni par TCI Europe)) utilisé comme réactif.

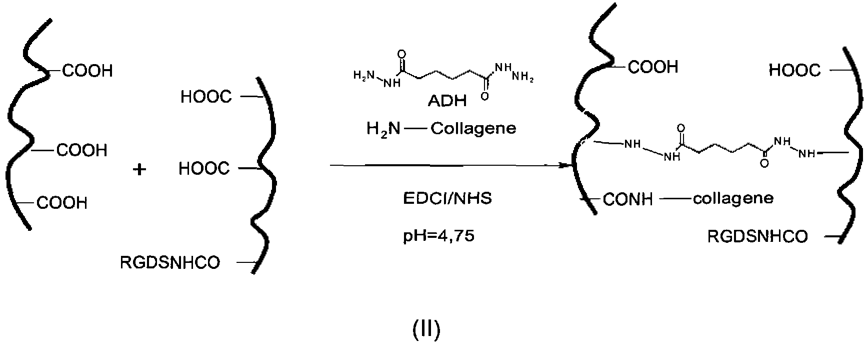

400 mg d'acide hyaluronique greffé par du RGDS, obtenu à l'étape 1, 0,787 mg de collagène de type I, 0,25 mg de collagène de type VI et 1,810 g l'acide adipique dihydrazide (ADH) ont été dissous dans 0,1 litre d'eau milliQ, et le pH de la solution a été ajusté à 4,75 par l'ajout d'HCl 0,1 M.

La concentration du HA dans le gel est égale à 4 mg/ml.

Le réactif EDCI (220 mg) a été dissout dans 2 ml d'eau milliQ sans purification préalable, puis a été ajouté au mélange réactionnel contenant de l'acide hyaluronique greffé par du RGDS, obtenu à l'étape 1, du collagène de type I, du collagène de type VI et de l'acide adipique dihydrazide.

Le mélange réactionnel a ensuite été laissé gélifier pendant 12 heures à température ambiante. La gélification est effectuée la première heure sous agitation douce puis le gel est laissé sans agitation, pendant 11 heures, à température ambiante, afin que le gel puisse se stabiliser.

Les hydrogels d'acide hyaluronique réticulés obtenus ont ensuite été purifiés par dialyse, i.e. équilibrés dans une solution de NaCl à 0,1 M pendant 2 jours, puis équilibrés dans un mélange eau / éthanol (3/1 en volume) pendant 2 jours, puis équilibrés dans l'eau milliQ pendant 2 jours afin d'éliminer les composés n'ayant pas réagi, tels que l'ADH.

Les hydrogels d'acide hyaluronique réticulés ont ensuite coulés dans des plaques de culture cellulaire congelés puis lyophilisés dans un lyophilisateur (Alpha 1-2 - performances: 2 kg de glace par 24 heures, T = -55 ° C) durant 24 heures.

Les hydrogels d'acide hyaluronique lyophilisés en forme cylindrique ont ensuite été stockés à -20 ° C.

Les hydrogels cylindriques ont ensuite compressés puis ont été chauffés à 100 ° C pendant 1 heure en utilisant un bain d'huile.

Les hydrogels ont ensuite été réhydratés (0,5 mg d'hydrogels lyophilisés introduits dans 100 mg d'une solution aqueuse d'acide hyaluronique à 2 g/L), transférés dans une plaque de culture cellulaire, congelés puis lyophilisés dans un lyophilisateur (Alpha 1-2 - performances: 2 kg de glace par 24 heures, T = -55 ° C) durant 24 heures.

Les hydrogels ont été ensuite exposés, à une dose de rayonnement gamma de 1 000 Gray afin de les stériliser.

Les hydrogels, se présentant sous la forme d'une poudre sèche, sont ensuite stockés à - 20°C sous conditions stériles, avant toute réhydratation et utilisation.

La présence des collagènes de type I et de type VI dans les hydrogels, i.e. dans la matrice, a été déterminée par spectroscopie infrarouge à transformée de Fourier (IRTF).

### 4.1.3. Caractérisation et propriétés physicochimiques des hydrogels

Les hydrogels ainsi obtenus ont ensuite été caractérisés et comparés à un hydrogel d'acide hyaluronique seul.

### ✔ Obtention d'un hydrogel d'acide hyaluronique seul

400 mg d'acide hyaluronique et 1,810 g l'acide adipique dihydrazide (ADH) ont été dissous dans 0,1 litre d'eau milliQ, et le pH de la solution a été ajusté à 4,75 par l'ajout d'HCl 0,1 M. La concentration du HA dans le gel est égale à 4 mg/ml. Le réactif EDCI (220 mg) a été dissout dans 2 ml d'eau milliQ sans purification préalable, puis a été ajouté au mélange réactionnel contenant de l'acide hyaluronique et de l'acide adipique dihydrazide.

Le mélange réactionnel a ensuite été laissé gélifier pendant 12 heures à température ambiante. La gélification est effectuée la première heure sous agitation douce puis le gel est laissé sans agitation, pendant 11 heures, à température ambiante, afin que le gel puisse se stabiliser.

Les hydrogels d'acide hyaluronique réticulés obtenus ont ensuite été purifiés par dialyse, i.e. équilibrés dans une solution de NaCl à 0,1 M pendant 2 jours, puis équilibrés dans un mélange eau / éthanol (3/1 en volume) pendant 2 jours, puis équilibrés dans l'eau milliQ pendant 2 jours afin d'éliminer les composés n'ayant pas réagi, tels que l'ADH.

Les hydrogels d'acide hyaluronique réticulés ont ensuite coulés dans des plaques de culture cellulaire congelés puis lyophilisés dans un lyophilisateur (Alpha 1-2 - performances: 2 kg de glace par 24 heures, T = -55 ° C) durant 24 heures.

Les hydrogels cylindriques ont ensuite compressés puis ont été chauffés à 100 ° C pendant 1 heure en utilisant un bain d'huile.

Les hydrogels ont ensuite été réhydratés (0,5 mg d'hydrogels lyophilisés introduits dans 100 mg d'une solution aqueuse d'acide hyaluronique à 2 g/L), transférés dans une plaque de culture cellulaire, congelés puis lyophilisés dans un lyophilisateur (Alpha 1-2 - performances: 2 kg de glace par 24 heures, T = -55 ° C) durant 24 heures.

Les hydrogels ont été ensuite exposés, à une dose de rayonnement gamma de 1 000 Gray afin de les stériliser.

Les hydrogels, se présentant sous la forme d'une poudre sèche, sont ensuite stockés à - 20°C sous conditions stériles, avant toute réhydratation et utilisation.

### ✔ Propriétés physicochimiques des hydrogels

Les mesures rhéologiques sont réalisées sur un rhéomètre HR2. Les tests consistent en un balayage en déformation (2 à 500 Pa) à fréquence constante (2Hz). Il permet de déterminer la zone linéaire de viscoélasticité et de connaître la déformation critique (γc) nécessaire pour briser le réseau d'hydrogel.

La détermination des propriétés physicochimiques et l'effet de l'incorporation des collagènes types I et VI dans la matrice a été obtenue par des mesures de taux de gonflement (Q) et des mesures rhéologiques de module élastique (G'), présentés dans le tableau 1 ci-après.

**Tableau 1**

| Hydrogel | Taux de gonflement (Q) [g/g] | Module élastique G' [KPa] |
|---|---|---|
| Acide hyaluronique seul | 50<Q<100 | 0,1 <G'<0,25 |
| Acide hyaluronique greffé par du RGDS et reticulé en présence de collagènes de type I et VI | 40<Q<90 | 0,1<G'<1,2 |

La comparaison des taux de gonflement des hydrogels avant et après l'incorporation des collagènes montre une diminution de taux de gonflement. Ce comportement est dû à la rigidification de la matrice par les collagènes. En effet les mesures rhéologiques montrent une augmentation du module G' suite à l'introduction des collagènes dans la matrice HA.

Les hydrogels ainsi obtenus ont aussi été observés en microscopie électronique à balayage. Ces derniers, comprenant du collagène de type I et de type VI (cf. figure 4C), présentent une structure différente d'un hydrogel comprenant de l'acide hyaluronique seul (cf. figure 4A).

### ✔ Quantification de l'accumulation intracellulaire de lipides lors de la culture cellulaire d'adipocytes au sein des hydrogels

La culture cellulaire d'adipocytes dans un hydrogel comprenant de l'acide hyaluronique greffé par du RGDS et réticulé en présence de collagènes de type I et VI a été étudiée et comparée à la culture cellulaire d'adipocytes dans un hydrogel comprenant de l'acide hyaluronique seul.

Pour ce faire, des cellules preadipocytes primaires humains, dénommées ci-après HWP ont été isolées à partir de tissu adipeux humain sous-cutané. Ces cellules préadipocytes sont adaptées à l'étude des mécanismes physiologiques qui contrôlent la prolifération, la différenciation et la fonction du tissu adipeux.

Le système de culture cellulaire PromoCell fournissant des préadipocytes avec des milieux de croissance optimisés et des milieux de différenciation et nutrition sans sérum a été utilisé.

Les cellules ont été cultivées dans 200 µL de milieu de croissance durant 48h, la différentiation en adipocytes a ensuite été induite durant 72h puis les cellules ont ensuite été cultivées en milieu de nutrition (200 µL) permettant la maturation des cellules en adipocytes. Un critère majeur de différenciation des adipocytes est leur capacité à accumuler et à stocker des acides gras (triglycérides). Le dosage des lipides intracellulaires (triglycérides) a ensuite été réalisé après 7j de nutrition, sur les adipocytes différenciés en culture dans des hydrogels constitués d'acide hyaluronique seul et dans des hydrogels comprenant de l'acide hyaluronique greffé par du RGDS et réticulé en présence de collagènes de type I et VI.

Afin d'évaluer l'effet des hydrogels sur la différenciation des pré-adipocytes, ou sur l'utilisation des lipides dans les adipocytes matures, le test Adipored Assay Reagent^{™} a été employé. Le réactif Adipored^{™} permet de marquer les triglycérides et ainsi de visualiser les vésicules lipidiques en microscopie de fluorescence, attestant la différenciation des cellules pré-adipocytes en adipocytes. Les cellules peu ou pas différenciées ne présentent pas ou peu de vésicules marquées par l'AdipoRed^{™}.

Après 7 jours de nutrition, 100 µL du milieu de nutrition ont été enlevés et une solution d' Adipored^{™} à 5% volumique en tampon phosphate salin PBS a été ajoutée. Les cellules ont ensuite été incubées à 37°C pendant 30 minutes. Les cellules ont ensuite été lavées par 200 µL de tampon PBS avant toute quantification.

La quantification des lipides intracellulaires marqués par l'Adipored^{™} au sein des matrices 3D a ensuite été effectuée par mesure de la fluorescence à une longueur d'onde de 572 nm et est présentée dans le tableau 2 ci-après.

**Tableau 2**

| Hydrogel | Cellules murines 3T3-L1 (unités de fluorescence arbitraire) | Cellules humaines HWP (unités de fluorescence arbitraire) |
|---|---|---|
| Acide hyaluronique seul | 12,52 ± 3,78 | 8,74 ± 0,42 |
| Acide hyaluronique greffé par du RGDS et reticulé en présence de collagènes de type I et VI | 24,01 ± 7,18 | 15,44 ± 4,55 |

Les tests montrent que la quantité des triglycérides a été doublée pour les cellules Murine (3T3-L1) et humaines (HWP) cultivées dans la matrice spécialisée comprenant de l'acide hyaluronique greffé par du RGDS et reticulé en présence de collagènes de type I et VI au regard des cellules 3T3-L1 et HWP cultivées dans une matrice comprenant de l'acide hyaluronique réticulé seul. Ceci indique une plus grande différenciation des pré-adipocytes en culture dans les hydrogels spécialisés selon l'invention.

### 4.2. Exemple 2 : Hydrogel pour culture cellulaire 3D d'hépatocytes

### 4.2.1. Modification du HA par greffage du (ARG-GLY-ASP-SER) RGDS

Dans certains modes de réalisation préférés, les acides hyaluroniques utilisés ont un poids moléculaire élevé (> 1x10⁶ Da).

Le schéma réactionnel du greffage du tetrapeptide RGDS sur l'acide hyaluronique est présenté ci-après (Schéma I).

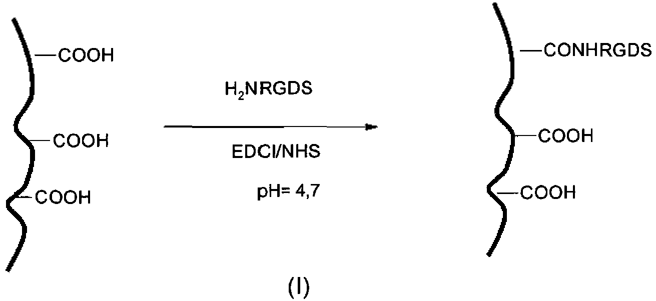

400 mg d'acide hyaluronique et 17 mg de tetrapeptide RGDS ont été ajoutés et dissous dans 0,1 litre d'eau.

5 mg de N-hydroxysuccinimide (NHS) et 6 mg d'ethyl(dimethylaminopropyl) carbodiimide (EDCI ou 1-éthyl-3-(3-diméthylaminopropyl)carbodiimide) ont été ajoutées à température ambiante, sans purification préalable, dans la solution aqueuse d'acide hyaluronique contenant le tetrapeptide RGDS précédemment préparée. Les ratios NHS/acide hyaluronique et d'EDCI/acide hyaluronique ont été ajustés afin d'obtenir des acides hyaluroniques greffés par du RGDS optimisés pour l'adhésion de cellules et pour la culture cellulaire.

Le pH, de la solution contenant le tetrapeptide RGDS et l'acide hyaluronique, a été fixé à 4,75 par l'ajout d'HCl 0,1 M. Le mélange a ensuite été agité pendant 12 heures à température ambiante afin d'obtenir de l'acide hyaluronique greffé par du RGDS.

La purification du HA greffé par du (ARG-GLY-ASP-SER) RGDS a été effectuée par filtration tangentielle.

L'acide hyaluronique greffé par du RGDS a ensuite été placé dans un récipient en plastique, congelé puis lyophilisé dans un lyophilisateur (Alpha 1-2 - performances: 2 kg de glace par 24 heures, T = -55 ° C) durant 24 heures.

Le taux de greffage de motif RGDS a été déterminé par ¹H-RMN (cf. figure 1) et est de l'ordre de 0,5%.

### 4.2.2. Modification du HA par greffage d'un motif galactosamine (H₂NGal)

Dans certains modes de réalisation préférés, les acides hyaluroniques utilisés ont un poids moléculaire élevé (> 1x10⁶ Da).

Le schéma réactionnel du greffage du motif H₂NGal sur l'acide hyaluronique est présenté ci-après dans le schéma III.

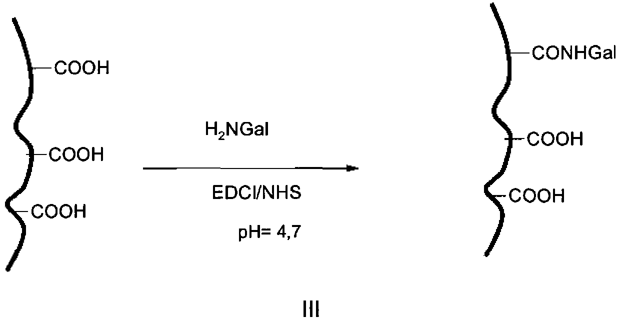

Une solution aqueuse de galactosamine H₂NGal (9 mM dans de l'eau) à température ambiante a été ajoutée progressivement dans une solution aqueuse d'acide hyaluronique (9 mM dans de l'eau) contenant du N-hydroxysuccinimide (NHS) et de l'ethyl(dimethylaminopropyl) carbodiimide (EDCI ou 1-éthyl-3-(3-diméthylaminopropyl)carbodiimide) à une concentration de 1.8 mM de NHS et 10 mM de EDCI dans de l'eau). Les ratios NHS/ acide hyaluronique et d'EDCl/acide hyaluronique ont été ajustés afin d'obtenir des acides hyaluroniques greffés par de la galactosamine optimisés pour l'adhérence de cellules et pour la culture cellulaire.

Le pH, de la solution contenant le motif galactosamine et l'acide hyaluronique, a été fixé à 4,75 par l'ajout d'HCl 0,1 M. Le mélange a ensuite été agité pendant 12 heures à température ambiante afin d'obtenir de l'acide hyaluronique greffé par de la galactosamine.

La purification du HA greffé par de la galactosamine a été effectuée par filtration tangentielle.

L'acide hyaluronique greffé par de la galactosamine a ensuite été placé dans un récipient en plastique, congelé puis lyophilisé dans un lyophilisateur (Alpha 1-2 - performances: 2 kg de glace par 24 heures, T = -55 ° C) durant environ 7 jours.

Le taux de greffage a été déterminé par ¹H-RMN (cf. figure 2) et est de l'ordre de 12%.

### 4.2.3. Réticulation des HA modifiés en présence de collagènes I et IV par l'ADH

Les hydrogels d'acides hyaluroniques modifiés ont été préparés comme le montre le schéma réactionnel suivant (IV), à partir de l'acide hyaluronique greffé par du RGDS, obtenu à l'étape 1, de l'acide hyaluronique greffé par de la galactosamine obtenu à l'étape précédente, du collagène de type I, du collagène de type IV, de l'acide adipique dihydrazide (ADH, fourni par TCI Europe) utilisé comme agent de réticulation et à partir d'EDCI (i.e. I-éthyl-3 [3- (diméthylamino) propyl] carbodiimide (EDCI, fourni par TCI Europe)) utilisé comme réactif.

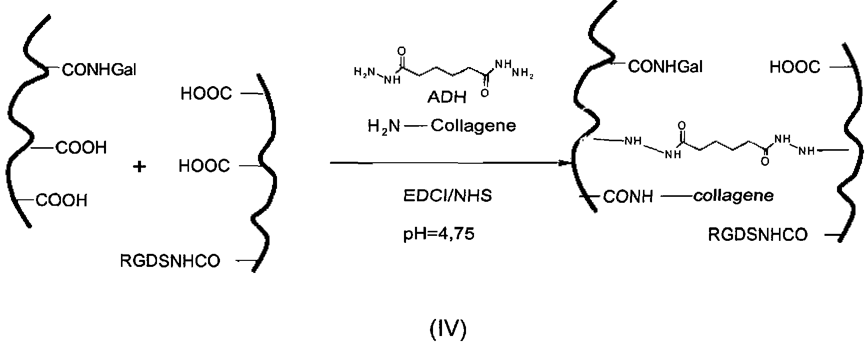

200 mg d'acide hyaluronique greffé par du RGDS, obtenu à l'étape 1, 200 mg d'acide hyaluronique greffé par de la galactosamine, obtenu à l'étape précédente, 0,8 g de collagène de type I, 0,2 g de collagène de type IV et 1,810 g d'acide adipique dihydrazide (ADH) ont été dissous dans 0,1 litre d'eau milliQ, et le pH de la solution a été ajusté à 4,75 par l'ajout d'HCl 0,1 M.

La concentration du HA dans le gel est égale à 4 mg/ml.

Le réactif EDCI (220 mg) a été dissout dans de 2 ml d'eau milliQ, puis a été ajouté au mélange réactionnel contenant de l'acide hyaluronique greffé par du RGDS, obtenu à l'étape 1, du collagène de type I, du collagène de type IV et de l'acide adipique dihydrazide.

Le mélange réactionnel est ensuite laissé gélifier pendant 12 heures à température ambiante. La gélification est effectuée la première heure sous agitation douce puis le gel est laissé sans agitation, pendant 11 heures, à température ambiante, afin que le gel puisse se stabiliser.

Les hydrogels d'acide hyaluronique réticulés obtenus ont ensuite été purifiés par dialyse, i.e. équilibrés dans une solution de NaCl à 0,1 M pendant 2 jours, puis équilibrés dans un mélange eau / éthanol (3/1 en volume) pendant 2 jours, puis équilibrés dans l'eau milliQ pendant 2 jours ou par diafiltration tangentielle afin d'éliminer les composés n'ayant pas réagi, tels que l'ADH.

Les hydrogels d'acide hyaluronique réticulés ont ensuite été coulés dans des plaques de culture cellulaire congelés puis lyophilisés dans un lyophilisateur (Alpha 1-2 - performances: 2 kg de glace par 24 heures, T = -55 ° C) durant 24 heures.

Les hydrogels d'acide hyaluronique lyophilisés en forme cylindrique ont ensuite été stockés à -20 ° C.

Les hydrogels cylindriques ont ensuite été compressés puis ont été chauffés à 100 ° C pendant 1 heure en utilisant un bain d'huile.

Les hydrogels ont ensuite été réhydratés. 0,5 mg d'hydrogels lyophilisés comprimés ont été introduits dans 100 mg d'une solution aqueuse d'acide hyaluronique à 2 g/L, puis ont été transférés dans une plaque de culture cellulaire, congelés puis lyophilisés dans un lyophilisateur (Alpha 1-2 - performances: 2 kg de glace par 24 heures, T = -55 ° C) durant 24 heures.

Les hydrogels lyophilisés ont été ensuite exposés aux rayonnements gamma, et ce, à une dose de rayonnement de 1 000 Gray afin de les stériliser.

Les hydrogels, se présentant sous la forme d'une poudre sèche, sont ensuite stockés à - 20°C sous conditions stériles, avant toute réhydratation et utilisation.

La présence des collagènes de type I et de type IV dans les hydrogels, i.e. dans la matrice, a été déterminée par infrarouge à transformation de fourrier (IRTF).

### 4.2.4. Caractérisation et propriétés physicochimiques des hydrogels

Les hydrogels ainsi obtenus ont ensuite été caractérisés et comparés à un hydrogel d'acide hyaluronique réticulé seul dénommé ci-après acide hyaluronique seul.

### ✔ Obtention d'un hydrogel d'acide hyaluronique seul

400 mg d'acide hyaluronique et 1,810 g l'acide adipique dihydrazide (ADH) ont été dissous dans 0,1 litre d'eau milliQ, et le pH de la solution a été ajusté à 4,75 par l'ajout d'HCl 0,1 M. La concentration du HA dans le gel est égale à 4 mg/ml. Le réactif EDCI (220 mg) a été dissout dans 2 ml d'eau milliQ sans purification préalable, puis a été ajouté au mélange réactionnel contenant de l'acide hyaluronique et de l'acide adipique dihydrazide.

Le mélange réactionnel a ensuite été laissé gélifier pendant 12 heures à température ambiante. La gélification est effectuée la première heure sous agitation douce puis le gel est laissé sans agitation, pendant 11 heures, à température ambiante, afin que le gel puisse se stabiliser.

Les hydrogels d'acide hyaluronique réticulés obtenus ont ensuite été purifiés par dialyse, i.e. équilibrés dans une solution de NaCl à 0,1 M pendant 2 jours, puis équilibrés dans un mélange eau / éthanol (3/1 en volume) pendant 2 jours, puis équilibrés dans l'eau milliQ pendant 2 jours afin d'éliminer les composés n'ayant pas réagi, tels que l'ADH.

Les hydrogels d'acide hyaluronique réticulés ont ensuite coulés dans des plaques de culture cellulaire congelés puis lyophilisés dans un lyophilisateur (Alpha 1-2 - performances: 2 kg de glace par 24 heures, T = -55 ° C) durant 24 heures.

Les hydrogels cylindriques ont ensuite compressés puis ont été chauffés à 100 ° C pendant 1 heure en utilisant un bain d'huile.

Les hydrogels ont ensuite été réhydratés (0,5 mg d'hydrogels lyophilisés introduits dans 100 mg d'une solution aqueuse d'acide hyaluronique à 2 g/L), transférés dans une plaque de culture cellulaire, congelés puis lyophilisés dans un lyophilisateur (Alpha 1-2 - performances: 2 kg de glace par 24 heures, T = -55 ° C) durant 24 heures.

Les hydrogels ont été ensuite exposés, à une dose de rayonnement gamma de 1 000 Gray afin de les stériliser.

Les hydrogels, se présentant sous la forme d'une poudre sèche, sont ensuite stockés à - 20°C sous conditions stériles, avant toute réhydratation et utilisation.

### ✔ Propriétés physicochimiques des hydrogels

La détermination des propriétés physicochimiques et l'effet de l'incorporation des collagènes types I et IV dans la matrice a été obtenue par des mesures de taux de gonflement (Q) et des mesures rhéologiques de module élastique (G'), présentés dans le tableau 3 ci-après.

**Tableau 3**

| hydrogel | Taux de gonflement (Q) [g/g] | Module élastique G' [KPa] |
|---|---|---|
| Acide hyaluronique seul | 50<Q<100 | 0,1<G'<0,25 |
| Acide hyaluronique modifié par du RGDS et de la galactosamine et reticulé en présence de collagènes de type I et IV | 45<Q<90 | 0,1<G'<2,5 |

La comparaison des taux de gonflement des hydrogels avant et après l'incorporation des collagènes, des motifs RGDS et le motif galactosamine montre une diminution de taux de gonflement. Ce comportement est dû à la rigidification de la matrice par ces additifs. En effet les mesures rhéologiques montrent une augmentation du module G' suite à l'introduction de ces différents additifs dans la matrice HA.

Les hydrogels ainsi obtenus ont aussi été observés en microscopie électronique à balayage (cf. figure 4B). Ces derniers, comprenant du collagène de type I et de type IV, présentent une structure différente d'un hydrogel comprenant de l'acide hyaluronique seul (cf. figure 4A).

### ✔ Expression de gènes au sein des hydrogels

La capacité des hydrogels à induire et/ou maintenir l'expression de gènes d'intérêt a été étudiée. Pour ce faire, les hydrogels lyophilisés et stérilisés ont été ensemencés avec des cellules d'intérêt, des cellules hépatocytes.

Les hydrogels ont été ensemencés avec 25 µl d'une solution de cellules d'intérêt, de cellules hépatocytes. Ont été ajoutés, 30 minutes après l'ensemencement de l'hydrogel, 175 µl de milieu de culture cellulaire exempt de cellules afin de favoriser la croissance cellulaire. Ensuite, toutes les 24 heures, la moitié (soit 100µL) de milieu de culture a été prélevée et remplacée par un milieu de culture neuf afin de favoriser la croissance cellulaire. La matrice cellulaire 3D a ensuite été caractérisée une semaine après la mise en culture cellulaire, i.e. l'ensemencement de l'hydrogel de manière à ce que la croissance des cellules d'intérêt soit suffisante. Une semaine après la mise en culture cellulaire, 150 µL du milieu de culture ont été extraits des hydrogels et 150 µL du réactif Trizol (Life Technologies) ont été introduits dans les hydrogels de manière à favoriser l'extraction des ARNm. Après homogénéisation, les hydrogels et le réactif sont prélevés puis centrifugés à 10 000 g pendant 10 minutes. L'ARNm, contenu dans le surnageant a ensuite été extrait.

Après extraction avec le réactif Trizol (Life Technologies), 100 ng d'ARNm total ont été utilisés pour une transcription inverse en utilisant une amorce de 6 nucléotides et le Kit transcriptase inverse MMLV (Life Technologies). Les réactions de PCR quantitatives ont été effectuées en utilisant le réactif Roche SYBER green 480 et un appareil LightCycler (Roche Diagnostic, Meylan, France). La spécificité d'amplification a été évaluée en déterminant la courbe de fusion du produit. Le programme suivant a été utilisé: une étape à 95° C pendant 10 min, puis 50 cycles de dénaturation à 95° C pendant 10 s, alignement à 65° C pendant 15 s et élongation à 72° C pendant 15 s.

La quantification des acides nucléiques a ensuite été effectuée de manière relative en comparant le niveau d'expression des gènes d'intérêt au niveau d'expression du gène de référence RPLP0 dont l'expression ne varie pas avec les conditions de culture.

La figure 5 montre, en unités arbitraires, l'expression des gènes UGT1A1, CYP1A1 et CYP3A4 des cellules hépatocytes primaires humaines pour un hydrogel d'acide hyaluronique seul et pour un hydrogel selon l'invention, i.e. matrice spécialisée comprenant de l'acide hyaluronique greffé par du RGDS et de la galactosamine et réticulé en présence de collagènes de type I et IV. L'augmentation de l'expression des gènes UGT1A1, CYP1A1 et CYP3A4 lorsque les hépatocytes sont cultivés dans des hydrogels spécialisés selon l'invention suggère un meilleur maintien des fonctions hépatocytaires.

La viabilité cellulaire de cellules hépatocytes présentes dans la matrice cellulaire 3D selon l'invention, comprenant notamment de l'acide hyaluronique greffé par du RGDS et de la galactosamine et réticulé en présence de collagènes de type I et IV a été étudiée et comparée à la viabilité cellulaire de cellules hépatocytes présentes dans une matrice comprenant de l'acide hyaluronique seul. Pour ce faire, des tests de toxicité in vitro sur des cellules HepG2 ont été réalisés. Les cellules HepG2 ont été cultivées pendant 7 jours puis elles ont été traitées pendant 24h avec un principe actif hépatotoxique, la chlorpromazine. La viabilité cellulaire a ensuite été quantifiée à l'aide du test WST-1 sur 6 réplicats et représentée en figure 6. Les cellules cultivées dans les hydrogels spécialisés selon l'invention montrent une résistance plus importante à la mort cellulaire induite par le principe actif hépatotoxique en comparaison des cellules cultivées dans les hydrogels comprenant de l'acide hyaluronique seul (cf. figure 6).

La concentration de chlorpromazine induisant 50% (IC50) et 10% (IC10) de mortalité cellulaire a été déterminée pour des cellules présentes dans la matrice cellulaire 3D selon l'invention, comprenant notamment de l'acide hyaluronique greffé par du RGDS et réticulé en présence de collagènes de type I et IV et comparée à la concentration de chlorpromazine induisant 50% (IC50) et 10% (IC10) de mortalité cellulaire pour des cellules hépatocytes présentes dans une matrice comprenant de l'acide hyaluronique seul.

L'IC50 et l'IC10 des cellules cultivées dans des gels spécialisés selon l'invention est deux fois plus importante que l'IC50 et l'IC10 des cellules cultivées dans des gels d'acide hyaluronique seul (cf. tableau 4). Ces résultats confirment les courbes de viabilité cellulaire observées en figure 6.

**Tableau 4**

| hydrogel | IC 50 (µM) | IC 10 (µM) |
|---|---|---|
| Acide hyaluronique seul | 33,44 | 17,18 |
| Acide hyaluronique modifié par du RGDS et de la galactosamine et réticulé en présence de collagènes de type I et IV | 71,55 | 35,51 |

### 4.3. Exemple 3 : Hydrogel pour culture cellulaire 3D à base de collagène de type I

La matrice hydrogel a été préparée en deux étapes.

### 4.3.1. Modification du HA par greffage du (ARG-GLY-ASP-SER) RGDS

Dans certains modes de réalisation préférés, les acides hyaluroniques utilisés ont un poids moléculaire élevé (> 1x10⁶ Da).

Le schéma réactionnel du greffage du tetrapeptide RGDS sur l'acide hyaluronique est présenté ci-après (Schéma I).

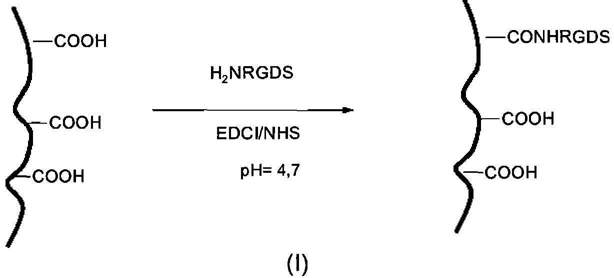

400 mg d'acide hyaluronique et 17 mg de tetrapeptide RGDS sont ajoutés et dissous dans 0,1 litre d'eau.

5 mg de N-hydroxysuccinimide (NHS) et 6 mg d'ethyl(dimethylaminopropyl) carbodiimide (EDCI ou 1-éthyl-3-(3-diméthylaminopropyl)carbodiimide) ont été ajoutées à température ambiante dans la solution aqueuse d'acide hyaluronique contenant le tetrapeptide RGDS précédemment préparée. Les ratios NHS/ acide hyaluronique et d'EDCI/acide hyaluronique ont été ajustés afin d'obtenir des acides hyaluroniques greffés par du RGDS optimisés pour l'adhérence de cellules et pour la culture cellulaire.

Le pH, de la solution contenant le tetrapeptide RGDS et l'acide hyaluronique, a été fixé à 4,75 par l'ajout d'HCl 0,1 M. Le mélange a ensuite été agité pendant 12 heures à température ambiante afin d'obtenir de l'acide hyaluronique greffé par du RGDS.

La purification du HA greffé par du (ARG-GLY-ASP-SER) RGDS a été effectuée par filtration tangentielle.

L'acide hyaluronique greffé par du RGDS a ensuite été placé dans un récipient en plastique, congelé puis lyophilisé dans un lyophilisateur (Alpha 1-2 - performances: 2 kg de glace par 24 heures, T = -55 ° C) durant 7 jours.

Le taux de greffage de motif RGDS a été déterminé par ¹H-RMN (cf. figure 1) et est de l'ordre de 1%.

### 4.3.2. Réticulation des HA modifiés en présence de collagènes I par l'ADH

Les hydrogels d'acides hyaluroniques modifiés ont été préparés comme le montre le schéma réactionnel suivant (II), à partir de l'acide hyaluronique greffé par du RGDS, obtenu à l'étape 1, du collagène de type I, de l'acide adipique dihydrazide (ADH, fourni par TCI Europe) utilisé comme agent de réticulation et à partir d'EDCI (i.e. I-éthyl-3 [3-(diméthylamino) propyl] carbodiimide, fourni par TCI Europe) utilisé comme réactif.

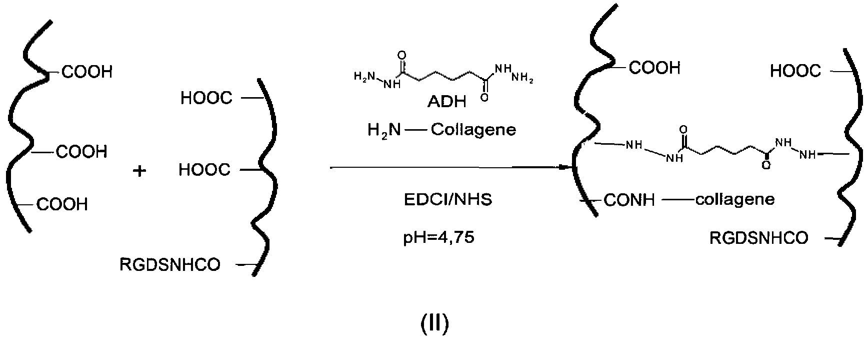

400 mg d'acide hyaluronique greffé par du RGDS, obtenu à l'étape 1, 0,8 mg de collagène de type I et 1,810 g d'acide adipique dihydrazide (ADH) ont été dissous dans 0,1 litre d'eau milliQ, et le pH de la solution a été ajusté à 4,75 par l'ajout d'HCl 0,1 M.

La concentration du HA dans le gel est égale à 4 mg/ml.

Le EDCI (220 mg) a été dissou dans 2 ml d'eau milliQ, puis a été ajouté au mélange réactionnel contenant de l'acide hyaluronique greffé par du RGDS, obtenu à l'étape 1, du collagène de type I et de l'acide adipique dihydrazide .

Le mélange réactionnel est ensuite laissé gélifier pendant 12 heures à température ambiante. La gélification est effectuée la première heure sous agitation douce puis le gel est laissé sans agitation, pendant 11 heures, à température ambiante, afin que le gel puisse se stabiliser.

Les hydrogels d'acide hyaluronique réticulés obtenus ont ensuite été purifiés par dialyse, i.e. équilibrés dans une solution de NaCl à 0,1 N pendant 2 jours, puis équilibrés dans un mélange eau / éthanol (3/1 en volume) pendant 2 jours, puis équilibrés dans l'eau milliQ pendant 2 jours ou par diafiltration tangentielle afin d'éliminer les composés n'ayant pas réagi, tels que l'ADH.

Les hydrogels d'acide hyaluronique réticulés ont ensuite été coulés dans des plaques de culture cellulaire congelés puis lyophilisés dans un lyophilisateur (Alpha 1-2 - performances: 2 kg de glace par 24 heures, T = -55 ° C) durant 24 heures.

Les hydrogels d'acide hyaluronique lyophilisés en forme cylindrique ont ensuite été stockés à -20 ° C.

Les hydrogels cylindriques ont ensuite été compressés puis ont été chauffés à 100 ° C pendant 1 heure en utilisant un bain d'huile.

Les hydrogels ainsi comprimés ont ensuite été réhydratés. 0,5 mg d'hydrogels lyophilisés comprimés ont été introduits dans 100 mg d'une solution aqueuse d'acide hyaluronique à 2 g/L, puis ont été transférés dans une plaque de culture cellulaire, congelés puis lyophilisés dans un lyophilisateur (Alpha 1-2 - performances: 2 kg de glace par 24 heures, T = -55 ° C) durant 24 heures.

Les hydrogels ont été ensuite exposés aux rayonnements gamma, et ce, à une dose de rayonnement de 1 000 Gray afin de les stériliser.

Les hydrogels, se présentant sous la forme lyophilisée, sont ensuite stockés à -20°C sous conditions stériles, avant toute réhydratation et utilisation.La présence de collagène de type I dans les hydrogels, i.e. dans la matrice, a été déterminée par infrarouge à transformation de fourrier (IRTF).

### 4.4. Utilisation des hydrogels comme milieu ou support de culture cellulaire

Les hydrogels ont été ensuite réhydratés pendant 24 heures dans le milieu de culture cellulaire RPMI 1640 (Eurobio) puis les hydrogels ont ensuite été lavés avec du PBS (D système, France R & D), afin de faciliter la migration et la colonisation des cellules dans les hydrogels. Les hydrogels ont ensuite été ensemencés avec des cellules d'intérêt, des cellules adipocytes.

Les hydrogels ont été ensemencés avec 50 µl d'une solution de cellules d'intérêt, de cellules adipocytes. Ont été ajoutés, 1 heure après l'ensemencement de l'hydrogel, 150 µl de milieu de culture cellulaire exempt de cellules afin de favoriser la croissance cellulaire. Ensuite, toutes les 48 heures, le milieu de culture a été extrait et remplacé par un milieu de culture propre afin de favoriser la croissance cellulaire. La matrice cellulaire 3D a ensuite été caractérisée une vingtaine de jours après la mise en culture cellulaire, i.e. l'ensemencement de l'hydrogel de manière à ce que la croissance cellulaire des cellules d'intérêt soit suffisante. La figure 3 montre la croissance cellulaire, observée par microscopie optique inversée, de 40 000 cellules préadipocytes humaines ensemencées sur les hydrogels pour culture cellulaire 3D adipocytes après 1 jour (figure 3 A), 4 jours (figure 3 B), 10 jours (figure 3 C) et 28 jours (figure 3 D) de culture cellulaire.

## Revendications

1. Procédé de fabrication d'un hydrogel lyophilisé comprenant au moins les étapes suivantes, dans lequel :
(a) on fait réagir une fonction amine du groupement peptidique ARG-GLY-ASP-SER (appelé « RGDS ») sur une fonction acide de l'acide hyaluronique, pour obtenir un premier acide hyaluronique au moins partiellement modifié, et
(b) on fait réagir ledit premier acide hyaluronique au moins partiellement modifié avec une solution aqueuse et/ou alcoolique d'un agent réticulant, de préférence de l'acide adipique dihydrazide (ADH), en présence d'au moins un collagène, et de préférence d'au moins un collagène de type I, pour obtenir un hydrogel réticulé,
(c) on lyophilise l'hydrogel réticulé obtenu à l'étape (b); et
(d) on stérilise l'hydrogel lyophilisé obtenu à l'étape (c).

2. Procédé de fabrication d'un hydrogel lyophilisé selon la revendication 1, dans lequel:
(a) on fait réagir une fonction amine du groupement peptidique ARG-GLY-ASP-SER (RGDS) sur une fonction acide de l'acide hyaluronique, pour obtenir un premier acide hyaluronique au moins partiellement modifié, et
(a') on fait réagir une fonction amine du galactosamine (H₂NGal) sur une fonction acide de l'acide hyaluronique, pour obtenir un second acide hyaluronique au moins partiellement modifié,
(b) on fait réagir ledit premier et/ou ledit second acide hyaluronique au moins partiellement modifié avec une solution aqueuse et/ou alcoolique d'un agent réticulant, de préférence de l'acide adipique dihydrazide (ADH) en présence d'au moins un collagène, et de préférence d'au moins un collagène de type I, pour obtenir un hydrogel réticulé,
(c) on lyophilise l'hydrogel réticulé obtenu à l'étape (b); et
(d) on stérilise l'hydrogel lyophilisé obtenu à l'étape (c).

3. Procédé selon la revendication 2, dans lequel à l'étape (a'), le taux de greffage des fonctions acides de l'acide hyaluronique est compris entre 0,5 et 25% molaire, de préférence compris entre 7 et 18%.

4. Procédé selon l'une quelconque des revendications précédentes, dans lequel à l'étape (a), le taux de greffage des fonctions acides de l'acide hyaluronique est compris entre 0,5 et 20 % molaire, de préférence 1%.

5. Procédé selon l'une quelconque des revendications précédentes, dans lequel le ratio molaire collagène / acide hyaluronique employé à l'étape b) est compris entre 0,1% et 15%.

6. Procédé selon l'une quelconque des revendications précédentes,, dans lequel l'étape (a), l'étape (a') et/ou l'étape (b) est effectuée à un pH compris entre 4,0 et 5,0, et de préférence à un pH compris entre 4,5 et 4,9 , et encore plus préférentiellement à un pH de 4,75.

7. Procédé selon l'une quelconque des revendications précédentes, dans lequel l'étape (a), l'étape (a') et/ou l'étape (b) est effectuée en présence d'EDCI et de NHS.

8. Procédé selon l'une quelconque des revendications précédentes, dans lequel les collagènes employés à l'étape (b) sont de type I et IV, sachant que de manière préférée :
- la proportion de collagène de type I est comprise entre 0,1% et 0,4% molaire, et / ou
- la proportion de collagène de type IV est comprise entre 0,05% et 0,15% molaire, et / ou
- le ratio molaire collagène de type I et IV / acide hyaluronique employé à l'étape (b) est compris entre 0,1% et 15%.

9. Procédé selon l'une quelconque des revendications 1 à 7, dans lequel les collagènes employés à l'étape (b) sont de type I et VI, sachant que de manière préférée le ratio molaire collagène de type I et VI / acide hyaluronique employé à l'étape (b) est compris entre 0,1% et 15%.

10. Procédé selon l'une quelconque des revendications précédentes dans lequel l'étape (d) de stérilisation de l'hydrogel obtenu à l'étape (c) est effectuée par exposition de l'hydrogel obtenu à l'étape (c) à des rayonnements ionisants, et plus particulièrement par exposition de l'hydrogel obtenu à l'étape (c) aux rayonnements gamma, de préférence à une dose comprise entre 1 000 Gray et 50 000 Gray, et encore plus préférentiellement à une dose comprise entre 1 000 Gray et 20 000 Gray.

11. Procédé selon les revendications précédentes dans lequel entre l'étape (c) de lyophilisation de l'hydrogel réticulé obtenu à l'étape (b) et l'étape (d) de stérilisation de l'hydrogel obtenu à l'étape (c) sont effectués :
- une étape de compression physique de l'hydrogel lyophilisé,
- et un traitement thermique de l'hydrogel, ce traitement thermique étant effectué, de préférence, par chauffage dans une gamme de température comprise entre 45°C et 110°C.

12. Produit susceptible d'être obtenu par le procédé selon l'une quelconque des revendications 1 à 11.

13. Hydrogel susceptible d'être obtenu à partir du produit selon la revendication 12 et à partir d'un liquide aqueux, ledit liquide aqueux comprenant possiblement du sérum et/ou un milieu de culture de cellules, la concentration de l'acide hyaluronique étant préférentiellement comprise entre 1 et 8 mg/mL.

14. Utilisation d'un produit ou d'un hydrogel selon l'une quelconque des revendications 12 à 13, dans un milieu pour la culture cellulaire 3D ou dans un milieu pour le transport de cellules.

15. Utilisation selon la revendication 14 pour la culture ou le transport de cellules de type adipocytes ou de cellules de type hépatocytes.

## Patentansprüche

1. Verfahren zur Herstellung eines gefriergetrockneten Hydrogels, umfassend mindestens die folgenden Schritte, wobei:
(a) eine Aminofunktion der ARG-GLY-ASP-SER-Peptidgruppe (genannt "RGDS") mit einer Säurefunktion der Hyaluronsäure umgesetzt wird, um eine erste Hyaluronsäure zu erhalten, die zumindest teilweise modifiziert ist, und
(b) die genannte erste Hyaluronsäure, die zumindest teilweise modifiziert ist, mit einer wässrigen und/oder alkoholischen Lösung eines Vernetzungsmittels, vorzugsweise Adipinsäuredihydrazid (ADH), in Gegenwart von mindestens einem Kollagen, und vorzugsweise von mindestens einem Kollagen vom Typ I, umgesetzt wird, um ein vernetztes Hydrogel zu erhalten,
(c) das in Schritt (b) erhaltene vernetzte Hydrogel gefriergetrocknet wird, und
(d) das in Schritt (c) erhaltene gefriergetrocknete Hydrogel sterilisiert wird.

2. Verfahren zur Herstellung eines gefriergetrockneten Hydrogels nach Anspruch 1, wobei:
(a) eine Aminofunktion der ARG-GLY-ASP-SER (RGDS)-Peptidgruppe mit einer Säurefunktion der Hyaluronsäure umgesetzt wird, um eine erste Hyaluronsäure zu erhalten, die zumindest teilweise modifiziert ist, und
(a') eine Aminofunktion von Galactosamin (H₂NGal) mit einer Säurefunktion der Hyaluronsäure umgesetzt wird, um eine zweite Hyaluronsäure zu erhalten, die zumindest teilweise modifiziert ist,
(b) die erste und/oder zweite Hyaluronsäure, die zumindest teilweise modifiziert ist, mit einer wässrigen und/oder alkoholischen Lösung eines Vernetzungsmittels, vorzugsweise Adipinsäuredihydrazid (ADH), in Gegenwart von mindestens einem Kollagen, und vorzugsweise von mindestens einem Kollagen vom Typ I, umgesetzt wird, um ein vernetztes Hydrogel zu erhalten,
(c) das in Schritt (b) erhaltene vernetzte Hydrogel gefriergerocknet wird; und
(d) das in Schritt (c) erhaltene gefriergetrocknete Hydrogel sterilisiert wird.

3. Verfahren nach Anspruch 2, wobei in Schritt (a') die Pfropfungsrate der Säurefunktionen der Hyaluronsäure zwischen 0,5 und 25 Mol-%, vorzugsweise zwischen 7 und 18 Mol-%, liegt.

4. Verfahren nach einem der vorhergehenden Ansprüche, wobei in Schritt (a) die Pfropfungsrate der Säurefunktionen der Hyaluronsäure zwischen 0,5 und 20 Mol-% liegt, und vorzugsweise 1% ist.

5. Verfahren nach einem der vorhergehenden Ansprüche, wobei das in Schritt b) verwendete molare Verhältnis von Kollagen zu Hyaluronsäure zwischen 0,1 % und 15 % liegt.

6. Verfahren nach einem der vorhergehenden Ansprüche, wobei Schritt (a), Schritt (a') und/oder Schritt (b) bei einem pH-Wert zwischen 4,0 und 5,0, vorzugsweise bei einem pH-Wert zwischen 4,5 und 4,9 und noch bevorzugter bei einem pH-Wert von 4,75 durchgeführt wird.

7. Verfahren nach einem der vorhergehenden Ansprüche, wobei Schritt (a), Schritt (a') und/oder Schritt (b) in Gegenwart von EDCI und NHS durchgeführt wird.

8. Verfahren nach einem der vorhergehenden Ansprüche, wobei die in Schritt (b) verwendeten Kollagene vom Typ I und IV sind, und wobei vorzugsweise:
- der Anteil an Kollagen vom Typ I zwischen 0,1 Mol-% und 0,4 Mol-% liegt, und/oder
- der Anteil an Kollagen vom Typ IV zwischen 0,05 Mol-% und 0,15 Mol-% liegt, und/oder
- das Molverhältnis von Kollagen vom Typ I und vom Typ IV zu Hyaluronsäure in Schritt (b) zwischen 0,1 % und 15 % liegt.

9. Verfahren nach einem der Ansprüche 1 bis 7, wobei es sich bei den in Schritt (b) verwendeten Kollagenen um solche vom Typ I und vom Typ VI handelt, wobei vorzugsweise das Molverhältnis von Kollagen vom Typ I und vom Typ VI zu Hyaluronsäure in Schritt (b) zwischen 0,1 % und 15 % liegt.

10. Verfahren nach einem der vorhergehenden Ansprüche, wobei der Schritt (d) der Sterilisation des in Schritt (c) erhaltenen Hydrogels durch Bestrahlung des in Schritt (c) erhaltenen Hydrogels mit ionisierender Strahlung und insbesondere durch Bestrahlung des in Schritt (c) erhaltenen Hydrogels mit Gammastrahlung durchgeführt wird, und vorzugsweise bei einer Dosis zwischen 1.000 Gray und 50.000 Gray, und noch bevorzugter zwischen 1.000 Gray und 20.000 Gray, durchgeführt wird.

11. Verfahren nach den vorhergehenden Ansprüchen, wobei zwischen dem Schritt (c) der Gefriertrocknung des in Schritt (b) erhaltenen vernetzten Hydrogels und dem Schritt (d) der Sterilisierung des in Schritt (c) erhaltenen Hydrogels folgende Schritte durchgeführt werden:
- ein physikalischer Kompressionsschritt des gefriergetrockneten Hydrogels,
- und eine Wärmebehandlung des Hydrogels, wobei diese Wärmebehandlung vorzugsweise durch Erhitzen in einem Temperaturbereich zwischen 45°C und 110°C durchgeführt wird.

12. Produkt, das durch das Verfahren gemäß einem der Ansprüche 1 bis 11 erhalten werden kann.

13. Hydrogel, das aus dem Produkt gemäß Anspruch 12 und aus einer wässrigen Flüssigkeit erhalten werden kann, wobei diese wässrige Flüssigkeit möglicherweise Serum und/oder ein Zellkulturmedium enthält, und wobei die Konzentration der Hyaluronsäure vorzugsweise zwischen 1 und 8 mg/ml liegt.

14. Verwendung eines Produkts oder Hydrogels nach einem der Ansprüche 12 bis 13 in einem Medium für die 3D-Zellkultur oder in einem Medium für den Zelltransport.

15. Verwendung gemäß Anspruch 14 für die Kultur oder den Transport von Adipozyten-oder Hepatozytenzellen.

## Claims

1. A method for manufacturing a freeze-dried hydrogel comprising at least the following steps, wherein:
(a) an amino function of the ARG-GLY-ASP-SER peptide group (called "RGDS") is reacted with an acid function of hyaluronic acid, to obtain a first hyaluronic acid that is at least partially modified, and
(b) said first hyaluronic acid, that is at least partially modified, is reacted with an aqueous and/or alcoholic solution of a crosslinking agent, preferably adipic acid dihydrazide (ADH), in the presence of at least one collagen, and preferably at least one type I collagen, to obtain a crosslinked hydrogel,
(c) the cross-linked hydrogel obtained in step (b) is freeze-dried; and
(d) the freeze-dried hydrogel obtained in step (c) is sterilized.

2. A method for manufacturing a freeze-dried hydrogel according to claim 1, wherein:
(a) an amino function of the ARG-GLY-ASP-SER (RGDS) peptide group is reacted with an acid function of hyaluronic acid, to obtain a first hyaluronic acid that is at least partially modified, and
(a') an amino function of galactosamine (H₂NGal) is reacted with an acid function of hyaluronic acid, to obtain a second hyaluronic acid that is at least partially modified,
(b) said first and/or second hyaluronic acid, that is at least partially modified, is reacted with an aqueous and/or alcoholic solution of a crosslinking agent, preferably adipic acid dihydrazide (ADH), in the presence of at least one collagen, and preferably at least one type I collagen, to obtain a crosslinked hydrogel,
(c) the cross-linked hydrogel obtained in step (b) is freeze-dried; and
(d) the freeze-dried hydrogel obtained in step (c) is sterilized.

3. A method according to claim 2, wherein in step (a'), the rate of grafting of the acid functions of hyaluronic acid is comprised between 0.5 and 25 molar %, preferably comprised between 7 and 18 molar %.

4. A method according to any one of the preceding claims, wherein in step (a), the rate of grafting of the acid functions of hyaluronic acid is comprised between 0.5 and 20 molar %, and is preferably 1 molar %.

5. A method according to any one of the preceding claims, wherein the collagen/hyaluronic acid molar ratio used in step b) is comprised between 0.1% and 15%.

6. A method according to any one of the preceding claims, wherein step (a), step (a') and/or step (b) is carried out at a pH comprised between 4.0 and 5.0, and preferably at a pH comprised between 4.5 and 4.9, and even more preferably at a pH of 4.75.

7. A method according to any one of the preceding claims, wherein step (a), step (a') and/or step (b) is carried out in the presence of EDCI and NHS.

8. A method according to any one of the preceding claims, wherein the collagens used in step (b) are of type I and IV, and wherein preferably:
- the proportion of type I collagen is comprised between 0.1 molar % and 0.4 molar %; and/or
- the proportion of type IV collagen is comprised between 0.05 molar % and 0.15 molar %, and/or
- the molar ratio of type I and IV collagen to hyaluronic acid used in step (b) is comprised between 0.1% and 15%.

9. A method according to any one of claims 1 to 7, wherein the collagens used in step (b) are of type I and VI, and wherein preferably the molar ratio of type I and VI collagen / hyaluronic acid used in step (b) is comprised between 0.1% and 15%.

10. A method according to any one of the preceding claims wherein step (d) of sterilizing the hydrogel obtained in step (c) is carried out by exposing the hydrogel obtained in step (c) to ionizing radiation, and more particularly by exposing the hydrogel obtained in step (c) to gamma radiation, preferably at a dose comprised between 1,000 Gray and 50,000 Gray, and preferably at a dose comprised between 1,000 Gray and 20,000 Gray.

11. A method according to the preceding claims, wherein between step (c) of freeze-drying the cross-linked hydrogel obtained in step (b) and step (d) of sterilizing the hydrogel obtained in step (c) are carried out:
- a physical compression step of the freeze-dried hydrogel,
- and a heat treatment of the hydrogel, this heat treatment being carried out, preferably, by heating in a temperature range comprised between 45°C and 110°C.

12. Product obtainable by the process according to any one of claims 1 to 11.

13. Hydrogel obtainable from the product according to claim 12 and from an aqueous liquid, said aqueous liquid possibly comprising serum and/or a cell culture medium, wherein wherein the concentration of hyaluronic acid is preferaby comprised between 1 and 8 mg/mL.

14. Use of a product or hydrogel according to any one of claims 12 to 13, in a medium for 3D cell culture or in a medium for cell transport.

15. Use according to claim 14 for the culture or transport of adipocyte-type cells or hepatocyte-type cells.
